# EUROPEAN PATENT APPLICATION

(11) **EP 3 081 640 A1**
(43) Date of publication of application: **19.10.2016**
(21) Application number: 15305545.4
(22) Date of filing: 13.04.2015
(51) Int. Cl.: C12N 9/12

(54) **MUTANT HUMAN DEOXYCYTIDINE KINASE**

(71) Applicant: Centre National de la Recherche Scientifique (C.N.R.S.), 75016 Paris (FR); Université de Strasbourg, 67000 Strasbourg (FR)
(72) Inventor: COULIBALY, Safiatou, 67100 Strasbourg (FR); ROSSOLILLO, Paola, 67000 Strasbourg (FR); NEGRONI, Matteo, 67000 Strasbourg (FR)
(74) Representative: Lavoix

(57) **Abstract**

The present invention concerns an isolated nucleic acid comprising a sequence encoding a mutant human deoxycytidine kinase (hdCK) capable of converting prodrugs, such as a nucleoside analogue, into cytotoxic drugs. The invention further refers to an isolated vector comprising said nucleic acid of the invention and an isolated host cell genetically engineered with said isolated vector. The invention also refers to a procedure for producing such polypeptides by recombinant techniques. The invention further refers to a pharmaceutical composition comprising an isolated nucleic acid, an expression vector, a host cell, or an isolated mutant hdCK of the invention. Their use as a medicament or their use as a medicament for the treatment of cancer or for the prevention of a viral infection is also part of the invention. The invention further refers to methods for utilizing such polypeptides for the treatment of malignancies and viral infections, methods of sensitizing cells to prodrugs, methods of gene therapy, methods of non-invasive nuclear imaging and methods of inhibiting pathogenic agents in a subject using said mutant hdCK.

SEQUENCE LISTING

<110>  CNRS PARIS
       UNIV STRASBOURG

<120>  HUMAN MUTANT DEOXYNUCLEOTIDE KINASE

<130>  BET 14P3137

<160>  13

<170>  PatentIn version 3.5

<210>  1
<211>  260
<212>  PRT
<213>  Homo sapiens

<400>  1

<210>  2
<211>  783
<212>  DNA
<213>  Homo sapiens

<400>  2
atggccaccc cgcccaagag aagctgcccg tctttctcag ccagctctga ggggacccgc      60

atcaagaaaa tctccatcga agggaacatc gctgcaggga agtcaacatt tgtgaatatc     120

cttaaacaat tgtgtgaaga ttgggaagtg gttcctgaac ctgttgccag atggtgcaat     180

gttcaaagta ctcaagatga atttgaggaa cttacaatgt ctcagaaaaa tggtgggaat     240

gttcttcaga tgatgtatga gaaacctgaa cgatggtctt ttaccttcca aacatatgcc     300

tgtctcagtc gaataagagc tcagcttgcc tctctgaatg gcaagctcaa agatgcagag     360

aaacctgtat tattttttga acgatctgtg tatagtgaca ggtatatttt tgcatctaat     420

ttgtatgaat ctgaatgcat gaatgagaca gagtggacaa tttatcaaga ctggcatgac     480

tggatgaata accaatttgg ccaaagcctt gaattggatg gaatcattta tcttcaagcc     540

actccagaga catgcttaca tagaatatat ttacggggaa gaaatgaaga gcaaggcatt     600

cctcttgaat atttagagaa gcttcattat aaacatgaaa gctggctcct gcataggaca     660

ctgaaaacca acttcgatta tcttcaagag gtgcctatct taacactgga tgttaatgaa     720

gactttaaag acaaatatga aagtctggtt gaaaaggtca aagagttttt gagtactttg     780

tga                                                                   783

<210>  3
<211>  260
<212>  PRT

<213>  Artificial Sequence

<220>
<223>  amino acid sequence of the so called "G12" full-length mutant
      hdCK

<400>  3

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Ser Leu Lys Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Lys Ser Met Val Glu Lys Val Lys Glu Phe
                245                 250                 255

Leu Ser Thr Leu
            260

<210>  4
<211>  783
<212>  DNA
<213>  Artificial Sequence

<220>
<223>  nucleotide sequence of the so-called "G12" full-length mutant
       hdCK

<400>  4
atggccaccc cgcccaagag aagctgcccg tctttctcag ccagctctga ggggacccgc      60

atcaagaaaa tctccatcga agggaacatc gctgcaggga agtcaacatt tgtgaatatc     120

cttaaacaat tgtgtgaaga ttgggaagtg gttcctgaac ctgttgccag atggtgcaat     180

gttcaaagta ctcaagatga atttgaggaa cttacaatgt ctcagaaaaa tggtgggaat     240

gttcttcaga tgatgtatga gaaacctgaa cgatggtctt ttaccttcca aacatatgcc     300

tgtctcagtc gaataagagc tcagcttgcc tctctgaatg gcaagctcaa agatgcagag     360

aaacctgtat tattttttga acgatctgtg tatagtgaca ggtatatttt tgcatctaat     420

ttgtatgaat ctgaatgcat gaatgagaca gagtggacaa tttatcaaga ctggcatgac     480

tggatgaata accaatttgg ccaaagcctt aaattggatg gaatcattta tcttcaagcc     540

actccagaga catgcttaca tagaatatat ttacggggaa gaaatgaaga gcaaggcatt     600

cctcttgaat atttagagaa gcttcattat aaacatgaaa gctggctcct gcataggaca     660

ctgaaaacca acttcgatta tcttcaagag gtgcctatct taacactgga tgttaatgaa     720

gactttaaag acaaatataa aagtatggtt gaaaaggtca aagagttttt gagtactttg     780

tga                                                                   783

<210>  5
<211>  260
<212>  PRT
<213>  Artificial Sequence

<220>
<223>  amino acid sequence of the so called "M36" full-length mutant
       hdCK

<400>  5

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Lys Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Lys Ser Met Val Glu Lys Val Lys Glu Phe
                245                 250                 255

Leu Ser Thr Leu
            260

<210>  6
<211>  783
<212>  DNA
<213>  Artificial Sequence

<220>
<223>  nucleotide sequence of the so-called "M36" full-length mutant
       hdCK

<400>  6
atggccaccc cgcccaagag aagctgcccg tctttctcag ccagctctga ggggacccgc      60

atcaagaaaa tctccatcga agggaacatc gctgcaggga agtcaacatt tgtgaatatc     120

cttaaacaat tgtgtgaaga ttgggaagtg gttcctgaac ctgttgccag atggtgcaat     180

gttcaaagta ctcaagatga atttgaggaa cttacaatgt ctcagaaaaa tggtggaaat     240

gttcttcaga tgatgtatga gaaacctgaa cgatggtctt ttaccttcca aacatatgcc     300

tgtctcagtc gaataagagc tcagcttgcc tctctgaatg gcaagctcaa agatgcagag     360

aaacctgtat tattttttga acgatctgtg tatagtgaca ggtatatttt tgcatctaat     420

ttgtatgaat ctgaatgcat gaatgagaca gagtggacaa tttatcaaga ctggcatgac     480

tggatgaata accaatttgg ccaaaacctt aaattggatg gaatcattta tcttcaagcc     540

actccagaga catgcttaca tagaatatat ttacggggaa gaaatgaaga gcaaggcatt     600

cctcttgaat atttagagaa gcttcattat aaacatgaaa gctggctcct gcataggaca     660

ctgaaaacca acttcgatta tcttcaagag gtgcctatct taacactgga tgttaatgaa     720

gactttaaag acaaatataa aagtatggtt gaaaaggtca aagagttttt gagtactttg     780

tga                                                                   783

<210>  7
<211>  260
<212>  PRT
<213>  Artificial Sequence

<220>
<223>   mutant hdCK having the mutation S169N

<400>  7

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp

35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Glu Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Glu Ser Leu Val Glu Lys Val Lys Glu Phe
                245                 250                 255

Leu Ser Thr Leu
            260

<210>  8
<211>  260
<212>  PRT
<213>  Artificial Sequence

<220>
<223>   mutant hdCK having the mutations S169N and E171K

<400>   8

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Lys Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu

225                 230                 235                 240

Asp Phe Lys Asp Lys Tyr Glu Ser Leu Val Glu Lys Val Lys Glu Phe
                245                 250                 255

Leu Ser Thr Leu
            260

<210>  9
<211>  260
<212>  PRT
<213>  Artificial Sequence

<220>
<223>   mutant hdCK having the mutations S169N and E247K

<400>  9

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Glu Leu Asp Gly Ile Ile
                165                 170                 175

Tyr Leu Gln Ala Thr Pro Glu Thr Cys Leu His Arg Ile Tyr Leu Arg
                180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Lys Ser Leu Val Glu Lys Val Lys Glu Phe
                245                 250                 255

Leu Ser Thr Leu
            260

<210>  10
<211>  260
<212>  PRT
<213>  Artificial Sequence

<220>
<223>   mutant hdCK having the mutations S169N and L249M

<400>  10

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Glu Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Glu Ser Met Val Glu Lys Val Lys Glu Phe
                245                 250                 255

Leu Ser Thr Leu
            260

<210>  11
<211>  260
<212>  PRT
<213>  Artificial Sequence

<220>
<223>   mutant hdCK having the mutations S169N, E171K and E247K

<400>  11

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Lys Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Lys Ser Leu Val Glu Lys Val Lys Glu Phe
                245                 250                 255

Leu Ser Thr Leu
            260

<210>  12
<211>  260
<212>  PRT
<213>  Artificial Sequence

<220>
<223>   mutant hdCK having the mutations S169N, E171K and L249M

<400>  12

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Lys Leu Asp Gly Ile Ile
                165                 170                 175

Tyr Leu Gln Ala Thr Pro Glu Thr Cys Leu His Arg Ile Tyr Leu Arg
            180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Glu Ser Met Val Glu Lys Val Lys Glu Phe
                245                 250                 255

Leu Ser Thr Leu
            260

<210>  13
<211>  260
<212>  PRT
<213>  Artificial Sequence

<220>
<223>   mutant hdCK having the mutations S169N, E247K and L249M

<400>  13

Met Ala Thr Pro Pro Lys Arg Ser Cys Pro Ser Phe Ser Ala Ser Ser
1               5                   10                  15

Glu Gly Thr Arg Ile Lys Lys Ile Ser Ile Glu Gly Asn Ile Ala Ala
            20                  25                  30

Gly Lys Ser Thr Phe Val Asn Ile Leu Lys Gln Leu Cys Glu Asp Trp
        35                  40                  45

Glu Val Val Pro Glu Pro Val Ala Arg Trp Cys Asn Val Gln Ser Thr
    50                  55                  60

Gln Asp Glu Phe Glu Glu Leu Thr Met Ser Gln Lys Asn Gly Gly Asn
65                  70                  75                  80

Val Leu Gln Met Met Tyr Glu Lys Pro Glu Arg Trp Ser Phe Thr Phe
                85                  90                  95

Gln Thr Tyr Ala Cys Leu Ser Arg Ile Arg Ala Gln Leu Ala Ser Leu
            100                 105                 110

Asn Gly Lys Leu Lys Asp Ala Glu Lys Pro Val Leu Phe Phe Glu Arg
        115                 120                 125

Ser Val Tyr Ser Asp Arg Tyr Ile Phe Ala Ser Asn Leu Tyr Glu Ser
    130                 135                 140

Glu Cys Met Asn Glu Thr Glu Trp Thr Ile Tyr Gln Asp Trp His Asp
145                 150                 155                 160

Trp Met Asn Asn Gln Phe Gly Gln Asn Leu Glu Leu Asp Gly Ile Ile
                165                 170                 175

Tyr Leu Gln Ala Thr Pro Glu Thr Cys Leu His Arg Ile Tyr Leu Arg

180                 185                 190

Gly Arg Asn Glu Glu Gln Gly Ile Pro Leu Glu Tyr Leu Glu Lys Leu
        195                 200                 205

His Tyr Lys His Glu Ser Trp Leu Leu His Arg Thr Leu Lys Thr Asn
    210                 215                 220

Phe Asp Tyr Leu Gln Glu Val Pro Ile Leu Thr Leu Asp Val Asn Glu
225                 230                 235                 240

Asp Phe Lys Asp Lys Tyr Lys Ser Met Val Glu Lys Val Lys Glu Phe
                245                 250                 255

Leu Ser Thr Leu
            260

## Description

The present invention concerns an isolated nucleic acid comprising a sequence encoding a mutant human deoxycytidine kinase (hdCK) capable of converting prodrugs, such as a nucleoside analogue, into cytotoxic drugs. The invention further refers to an isolated vector comprising said nucleic acid of the invention and an isolated host cell genetically engineered with said isolated vector. The invention also refers to a procedure for producing such polypeptides by recombinant techniques. The invention further refers to a pharmaceutical composition comprising an isolated nucleic acid, an expression vector, a host cell, or an isolated mutant hdCK of the invention. Their use as a medicament or their use as a medicament for the treatment of cancer or for the prevention of a viral infection is also part of the invention. The invention further refers to methods for utilizing such polypeptides for the treatment of malignancies and viral infections, methods of sensitizing cells to prodrugs, methods of gene therapy, methods of non-invasive nuclear imaging and methods of inhibiting pathogenic agents in a subject using said mutant hdCK.

Inducing death of cancer cells through gene therapy is the Holy Grail in the field of biotechnological applications in medicine. The main obstacle to reach this goal is constituted by the possibility of targeting specifically cancer cells for the delivery of the transgene that will induce cell death. A second important problem hampering such approaches is constituted by the efficiency of the transgene to use in order to induce cell death. Two approaches can be followed, one aimed at inducing constitutively cell death by the simple presence of the protein encoded by the transgene, as it is the case for genes that drive cells to apoptosis, or induce cell death by their toxicity; the second approach refers to genes sensitizing cells to the exposure of a chemical compound. Genes sensitizing cells to the exposure of anti-cancer chemicals are the candidates for this latter approach.

Such a potent inducible suicide gene might also be used as a safety gene allowing negative selection of cells transplanted in gene therapy approaches in case said transplanted cells undergo uncontrolled proliferation or induce severe side effects including, for example, severe immune reactions after engraftment.

So far, the suicide gene employed has been the thymidine kinase of herpes simplex virus (hsTK), used in combination with gancyclovir treatment. Being of viral origin, though, hsTK presents the drawback of inducing a graft versus host reaction that limits the efficiency of the therapeutic intervention.

Physiologically, the human deoxycytidine kinase (hdCK) is a crucial enzyme involved in the salvage pathway that converts recycled deoxyribonucleosides into deoxyribonucleoside triphosphate (dNTP) (Reichard P, 1988 Annu Rev Biochem). In analogy, hdCK is also the enzyme catalyzing the rate-limiting phosphorylation step for the activation of various deoxycytidine analogs (dCa) used in clinical treatments of various cancers. Enhanced sensitivity to dCa has also been shown to be induced through gene transfer in cancer cells of extra copies of the wild-type hdCK gene. Indeed, a correlation between the level of expression of hdCK and sensitivity to treatment with dCa has been observed in patients treated for hairy-cell leukemia and chronic lymphocytic leukemia (Kawasaki H et al., Blood 1993, 81, 567-601) or in various breast cancer cell lines (Geutjes, EJ et al., Breast Cancer Res Treat. 2012, 131(3): 809-818). DCK deficiency is also linked to resistance to other dCa as it has been shown for AraC (Lee et al. Radiation Oncology 2013, 8: 223). Thereby, the possibility of foreseeing gene therapy approaches based on the insertion of extra copies of this gene in cancer cells appears promising and the possibility of generating variants of the hdCK characterized by a specific improved phosphorylation of the dCa is a major goal in the field.

Rational attempts to design mutants of hdCK with the desired properties have been made in the past, based either on the structural information (Sabini E. et al. Nat Struct Biol. 2003, 10(7): 513-519) or on the effect of post-translational modifications of this protein observed to enhance its enzymatic activity *in vivo* (McSorley T. et al. FEBS Lett. 2008 582: 720-724 and Hazra S. et al., Biochemistry 2011 50: 2870-2880). This way, variants with an improved efficiency of phosphorylation for gemcitabine had been obtained, albeit in parallel to a more important enhancement of phosphorylation of the natural substrate deoxycytidine (dC). This was expected to result in no improved sensitization to dCa treatments since phosphorylation of the natural substrate was expected to be favored. This was indeed documented when the triple mutant described by Sabini and colleagues was introduced in the uterine sarcoma Messa10K cells and no sensitization to gemcitabine was observed (Rossolillo, P. et al. PLoS Genet. 2012 Aug; 8(8):e1002904).

The inventors of the present invention recently discovered a hdCK mutant, called G12, comprising three mutations, namely Glu171Lys, Glu247Lys and Leu249Met, which has been described in Rossolillo, P. et al. (PLoS Genet. 2012 Aug; 8(8):e1002904) and in the international patent application WO2011/058081.

G12 induces a 300-fold sensitization to gemcitabine in cells originally resistant to this drug (Messa10K), an effect 60 times stronger than the one induced with the wild-type enzyme. This effect could be temptatively correlated to an almost complete loss of ability, with respect to the wild type enzyme, to phosphorylate dC, while the efficiency of phosphorylation of gemcitabine remained almost unaltered (Rossolillo, P. et al. PLoS Genet. 2012 Aug; 8(8):e1002904).

In context of the present invention, the inventors discovered mutants that induce an improved sensitization of cells to gemcitabine with respect to G12 or wild-type hdCK. The mutant M36, for example, contains the mutations Ser169Asn, Glu171Lys, Glu247Lys and Leu249Met, and induces a 900-fold sensitization to gemcitabine in cells originally resistant to this drug. Furthermore, M36 induces a sensitization of cells to cytarabine (AraC) by a factor of 10,000 with an IC50 that drops from 10 mM to 1 µM. These results are surprising and unexpected, in particular because the additional amino acid mutation Ser169Asn is located in the vicinity of one of the mutations present in G12 localized in a region of the protein that does not have an important role in the kinase function according to structural data.

### Summary of the invention

The present invention therefore relates to an isolated nucleic acid comprising a sequence encoding a mutant human deoxycytidine kinase (hdCK), wherein said mutant hdCK comprises a polypeptide sequence which differs from the wild-type hdCK of sequence SEQ ID NO: 1 by at least one mutation, wherein the at least one mutation is at the amino acid position S169 in comparison to the amino acid sequence SEQ ID NO: 1

The present invention also relates to an isolated vector comprising the nucleic acid of the invention.

In a further aspect, the Invention refers to an isolated host cell genetically engineered with said isolated vector.

The present invention further relates to a process for producing a mutant hdCK comprising *in vitro* culturing a host cell of the invention and recovering the expressed mutant hdCK from the cultured host cells and/or culture medium.

The invention further concerns an isolated mutant hdCK comprising a polypeptide sequence which differs from wild-type hdCK of sequence SEQ ID NO: 1 by at least one mutation, wherein the at least one mutation is at the amino acid position S169 in comparison to the amino acid sequence SEQ ID NO: 1.

In a further aspect of the invention, the invention refers to a pharmaceutical composition comprising an isolated nucleic acid, an expression vector, a host cell or an isolated mutant hdCK according to the invention and optionally a pharmaceutically acceptable carrier or diluent.

The invention also concerns an isolated nucleic acid, an expression vector, a host cell, an isolated mutant hdCK of the invention, or a pharmaceutical composition thereof for use as a medicament.

In one embodiment, the medicament is for use for the treatment of cancer or for the prevention of a viral infection.

In a further aspect, the invention refers to a kit comprising a pharmaceutical composition of the invention and a nucleoside analogue.

The invention also concerns a method of sensitizing a cell to a nucleoside analogue prodrug, which method comprises the steps of:
(i) transfecting or transducing cells with a nucleic sequence of the invention, or with an expression vector of the invention.

The invention further refers to a method of inhibiting a pathogenic agent in a subject, which method comprises administering to said subject a nucleic acid or an expression vector of the invention.

In one aspect of the invention, the invention further refers to an method of phosphorylating a nucleoside or nucleoside analogue comprising the steps of:
(i) subjecting the nucleoside or nucleoside analogue to the action of the mutant hdCK of the invention, and
ii) recovering the phosphorylated nucleoside or nucleoside analogue.

The invention also refers to a method for the treatment of a patient having need of a human mutant deoxycytidine kinase comprising: administering to the patient a therapeutically effective amount of the isolated nucleic acid, a vector, an isolated human mutant deoxycytidine kinase, a host cell or a pharmaceutical composition according to the invention.

The invention further refers to a method of gene therapy in a subject, which method comprises the steps of:
(i) transfecting or transducing cells of a subject with a nucleic acid comprising mutant hdCK, a further transgene and a reporter gene, or transfecting or transducing cells of a subject with a nucleic acid comprising mutant hdCK and one nucleic acid comprising mutant hdCK, wherein one of the nucleic acids further comprises a reporter gene ; and
ii) monitoring if said cells undergo uncontrolled proliferation in the subject, and
iii) administering the subject with a nucleoside analogue prodrug, if said transfected or tranduced cells undergo uncontrolled proliferation.

### Description of the invention

### Definitions

"Deoxvcvtidine kinase" also known as "dCK" or "DCK" refers to an enzyme that catalyzes reversibly the phosphorylation of several deoxyribonucleosides and their nucleoside analogs. DCK transfers a phosphoryl group from ATP (or other triphosphorylated nucleotides) to deoxyribonucleosides. In particular, dCK phosphorylates deoxycytidine, deoxyadenosine and deoxyguanosine. Native dCK has been shown to localize in the cytoplasm. DCK is required for the phosphorylation of numerous nucleoside analogs used in chemotherapy, including araC (1-β-D-arabinofuranosylcytosine; Cytarabine), gemcitabine (2',2'-difluorodeoxycytidine; dFdC), fludarabine (2-fluoro-9-β-D-arabinosyladenine; FaraA) and cladribine (2-chlorodeoxyadenosine, 2CdA) (Van Rompay et at., 2000, Pharmacol. Ther. 87:189-98). Therefore, the activity of dCK is one of the factors that determines the sensitivity of cancer cells (including leukemias and certain solid tumors) to deoxynucleoside toxicity and therapy (Stegmann et al., 1995, Blood 85:1188-94; Lotfi et al., 1999, Clin. Cancer Res. 5:2438-44; Kakihara et al., 1998, Leuk. Lymphoma 31:405-9; Bergman et al., 1999, Biochem. Pharmacol. 57:397-406; Goan et al., 1999, Cancer Res. 59:4204-7).

There is a direct correlation between dCK enzymatic activity in tumor cell lines and the sensitivity of those cell lines to the toxicity of nucleoside analog chemotherapeutic prodrugs (Hapke et al., 1996, Cancer Res. 56:2343-7). Cells lacking dCK activity are resistant to a variety of drugs, including Ara-C, cladribine, fludarabine and gemcitabine (Owens et al., 1992, Cancer Res. 52:2389-93; Ruiz van Haperen et al., 1994, Cancer Res. 54:4138-43) and drug sensitivity to Ara-C can be restored by expressing functional dCK protein in cells that do not express this enzyme natively or in which only mutationally-inactivated forms thereof are expressed (Stegmann et al., 1995, Blood 85:1188-94). Moreover, *in vivo* studies conducted on animal tumors using gemcitabine showed that increased dCK activity, mediated by dCK gene transfer, resulted in enhanced accumulation and prolonged elimination kinetics of gemcitabine triphosphate, and ultimately, to a better tumor response to the prodrug (Blackstock et a.l, 2001, Clin. Cancer Res. 7:3263-8).

The deoxycytidine kinase in context of the invention is of human origin. The human deoxycytidine kinase gene is located on chromosome 4 and comprises 7 exons.

In one embodiment, the nucleotide sequence of the human wild-type deoxycytidine kinase comprises the nucleotide sequence of SEQ ID NO: 2. The nucleotide sequence SEQ ID NO: 2 corresponds to the nucleotides 41 to 823 of the human deoxycytidine kinase mRNA sequence as available from the GenBank database under accession number JF806429.1, as available on January 26, 2015.

In one embodiment, the amino acid sequence of the wild-type human deoxycytidine kinase comprises the amino acid sequence SEQ ID NO: 1. The amino acid sequence SEQ ID NO: 1 corresponds to the sequence of access number NP_000779.1 (NCBI database), as available on January 26, 2015.

A "deoxyribonucleoside" is a compound composed of deoxyribose and either a purine or a pyrimidine. Deoxyribonucleosides known to the skilled in the art are for example deoxyadenosine, deoxyguanosine, thymidine, deoxyuridine, deoxycytidine, deoxyinosine, deoxyxanthosine.

The deoxycytidine kinase in context of the invention phosphorylates deoxyribonucleosides and/or deoxyribonucleoside analogs.

In one embodiment, the deoxyribonucleosides in context of the invention are selected from the group consisting of deoxycytidine (dC), deoxyadenosine (dA) and deoxyguanosine (dG), preferably deoxycytidine (dC).

A "deoxyribonucleoside" may also be referred to as "nucleoside" and accordingly "deoxyribonucleoside analogue" may also be referred to as "nucleoside analogue".

A "nucleoside analogue" or "nucleoside analogue prodrug" herein refers to a compound comprising a deoxyribonucleoside structure, which compound is substituted in relation to a naturally occurring deoxyribonucleoside either on the deoxyribose part, or in the purine or pyrimidine ring. A nucleoside analogue is essentially non-toxic in its non-phosphorylated (nucleoside) state. Analogs of the naturally occurring nucleosides are usually administered as prodrugs, e.g. unphosphorylated, as the omission of the negative charges from the phosphate groups allows effective transport of the analog into the cell. Once prodrugs are converted into a potent cytotoxic metabolite, they inhibit or disrupt DNA synthesis. The treated cells subsequently die via necrotic or apoptotic pathways.

Nucleoside analogues in context of the invention are for example deoxyadenosine, deoxycytidine, deoxyguanosine, deoxythymidine, and deoxyuridine analogues and purine analogues, preferred nucleoside analogues are deoxycytidine, deoxyadenosine and deoxyguanosine and purine analogues, more preferably deoxycytidine analogues.

A purine analogue is for example fludarabine or cladribine.

A deoxycytidine analogue is for example gemcitabine, cytarabine (Ara-C), emtricitabine (FTC), lamivudine (3TC) or zalcitabine (ddC), preferably gemcitabine and cytarabine.

A deoxyadenosine analogue is for example didanosine (ddl) or vidarabine.

A deoxyguanosine analogue is for example entecavir.

A thymidine analogue is for example stavudine (d4T), telbivudine or zidovudine (azidothymidine, or AZT).

A deoxyuridine analogue is for example idoxuridine and trifluridine.

Accordingly, in one embodiment, the nucleoside analogue is selected from the group consisting of fludarabine, cladribine, gemcitabine, cytarabine, emtricitabine (FTC), lamivudine (3TC), zalcitabine (ddC), didanosine (ddl), vidarabine, entecavir, stavudine (d4T), telbivudine, zidovudine (azidothymidine, or AZT), idoxuridine and trifluridine, preferably cytarabine, emtricitabine (FTC), lamivudine (3TC) and zalcitabine (ddC), didanosine (ddl), vidarabine and entecavir, preferably fludarabine, cladribine, gemcitabine, cytarabine (Ara-C), lamivudine (3TC) and zalcitabine (ddC), more preferably the deoxycytidine analogues gemcitabine and cytarabine (Ara-C).

"Suicide gene therapy" refers to the transfection or tranduction of target cells with the so-called suicide gene to sensitize target cells towards a prodrug.

The term "transfection" refers to the process of introducing nucleic acids into cells. In particular "transfection" herein refers to introducing nucleic acids into eukaryotic cells by non-viral methods. Different transfection methods are known to the skilled in the art and include methods using calcium phosphate, electroporation, cell squeezing or liposomes.

The term "transduction" herein refers to the process whereby foreign DNA is introduced into another cell via a viral vector. Viral vectors are as defined in the section vectors.

The term "isolated" in reference to a biological component (such as a nucleic acid, a vector or a protein) refers to a biological component that has been substantially separated or purified away from other biological components in the cell of the organism, or the organism itself, in which the component naturally occurs, such as other chromosomal and extra-chromosomal DNA and RNA, proteins, cells, and organelles. "Isolated nucleic acids" or "isolated vectors" include nucleic acid molecules purified by standard purification methods. These terms also encompass nucleic acids and vectors prepared by amplification and/or cloning, as well as chemically synthesized nucleic acids and vectors.

Throughout the instant application, the term "and/or" is a grammatical conjunction that is to be interpreted as encompassing that one or more of the cases it connects may occur. For example, the wording "amplification and/or cloning" in the phrase "nucleic acids and vectors prepared by amplification and/or cloning" indicates that the nucleic acids and vectors in context of the invention may be prepared by amplification or cloning or amplification and cloning.

Reference herein to polypeptides and nucleic acids includes both the particular amino acid sequences and nucleic acid sequences disclosed herein, and variants of said sequences.

Variant proteins may be naturally occurring variants, such as splice variants, alleles and isoforms, or they may be produced by recombinant means. Variations in amino acid sequence may be introduced by substitution, deletion or insertion of one or more codons into the nucleic acid sequence encoding the protein that results in a change in the amino acid sequence of the protein. Optionally the variation is by substitution of 1, 2, 3, 4, 5, 6, 7, 8, 9, 10, 11, 12, 13, 14, 15, 16, 17, 18, 19, 20 or more amino acids with any other amino acid in the protein. Additionally or alternatively, the variation may be by addition or deletion of 1, 2, 3, 4, 5, 6, 7, 8, 9, 10, 11, 12, 13, 14, 15, 16, 17, 18, 19, 20 or more amino acids within the protein.

Variant nucleic acid sequences include sequences capable of specifically hybridizing to the sequence SEQ ID NOs: 2, 4 or SEQ ID NO: 6 under moderate or high stringency conditions. Stringent conditions or high stringency conditions may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C. Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C.

Variant proteins may include proteins that have at least about 80% amino acid sequence identity with a polypeptide sequence disclosed herein. Preferably, a variant protein will have at least about 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a polypeptide sequence as disclosed herein. Amino acid sequence identity is defined as the percentage of amino acid residues in the variant sequence that are identical with the amino acid residues in the reference sequence, after aligning the sequences and introducing gaps, if necessary, to achieve the maximum percent sequence identity, and not considering any conservative substitutions as part of the sequence identity. Sequence identity may be determined over the full length of both variant and reference amino acid sequences.

Variant nucleic acid sequences may include nucleic acid sequences that have at least about 80% amino acid sequence identity with a nucleic acid sequence disclosed herein. Preferably, a variant nucleic acid sequences will have at least about 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a nucleic acid sequence or a fragment of a nucleic acid sequence as disclosed herein. Nucleic acid acid sequence identity is defined as the percentage of nucleic acids in the variant sequence that are identical with the nucleic acids in the reference sequence, after aligning the sequences and introducing gaps, if necessary, to achieve the maximum percent sequence identity, and not considering any conservative substitutions as part of the sequence identity. Sequence identity may be determined over the full length of both variant and reference nucleic acid sequences.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

In the context of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise mutations such as deletions, insertions and/or substitutions compared to the reference sequence. In case of deletions, insertions and/or substitutions, the protein consisting of an amino acid sequence at least 80%, 85%, 90%, 95%, 96%, 97%, 98% or 99% identical to a reference sequence may correspond to a homologous sequence derived from another species than the reference sequence.

Amino acid substitutions may be conservative or non-conservative. Preferably, substitutions are conservative substitutions, in which one amino acid is substituted for another amino acid with similar structural and/or chemical properties. The substitution preferably corresponds to a conservative substitution as indicated in the table below.

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

### Polynucleotides

The present invention particularly relates to an isolated nucleic acid comprising a sequence encoding a mutant human deoxycytidine kinase (hdCK), wherein said mutant hdCK comprises a polypeptide sequence which differs from wild-type hdCK of sequence SEQ ID NO: 1 by at least one mutation, wherein said at least one mutation is at the amino acid position S169 in comparison to said amino acid sequence SEQ ID NO: 1. The nucleic acid of the invention encodes a mutant hdCK, this mutant hdCK may also be referred to as the polypeptide or protein of the invention.

A "polynucleotide" or "nucleic acid" herein refers to the phosphate ester polymeric form of ribonucleosides (also called "RNA molecule"), deoxyribonucleosides (also called "DNA molecule") or any phosphoester analogs thereof, such as phosphorothioates and thioesters, in either single stranded form or a double-stranded form. In one embodiment, the polynucleotide is selected from the group constituted of DNA, RNA, genomic DNA and cDNA.

In one embodiment, said mutant hdCK comprises a polypeptide sequence which differs from wild-type hdCK of sequence SEQ ID NO: 1 by at least one further mutation, wherein the at least one further mutation is selected at the position E171, E247 or L249, in comparison to the amino acid sequence SEQ ID NO: 1.

In one embodiment, the at least one further mutation refers to at least one, two or at least three further amino acid mutations.

Accordingly, in a further embodiment, the isolated nucleic acid comprises a sequence encoding a mutant human deoxycytidine kinase (hdCK), wherein said mutant hdCK comprises a polypeptide sequence which differs from wild-type hdCK of sequence SEQ ID NO: 1 by at least two mutations, wherein the at least two mutations are
a) at the amino acid positions S169 and E171, or
b) at the amino acid positions S169 and L249, or
c) at the amino acid positions S169 and L249.

Accordingly, in a further embodiment, said mutant hdCK comprises a polypeptide sequence which differs from wild-type hdCK of sequence SEQ ID NO: 1 by at least three mutations, wherein the at least three mutations are
a) at the amino acid positions S169, E171 and E247, or
b) at the amino acid positions S169, E171 and L249, or
c) at the amino acid positions S169, E247 and L249.

Accordingly, in a further embodiment, said mutant hdCK comprises a polypeptide sequence which differs from wild-type hdCK of sequence SEQ ID NO: 1 by at least four mutations, wherein the at least four mutations are at the amino acid positions S169, E171, E247 and L249.

In one embodiment, the mutation at position S169 is selected from the substitutions S169N, S169T, and S169Q, preferably S169N.

In one embodiment, the mutation at position E171 is selected from the substitutions E171 K, and E171 R, preferably E171 K.

In one embodiment, the mutation at position E247 is selected from the substitutions E247K, and E247R, preferably E247K.

In one embodiment, the mutation at position L249 is selected from the substitutions L249M, L249F, L249Y, L249I and L249W, preferably L249M.

In a further embodiment, said mutant hdCK is the so called mutant "M36" and comprises a polypeptide sequence which differs from wild-type hdCK of sequence SEQ ID NO: 1 by at least four mutations, wherein the at least four mutations are S169N, E171 K, E247K and L249M.

In one embodiment, the nucleotide acid comprises a sequence encoding a mutant hdCK of SEQ ID NO: 5, 7, 8, 9, 10, 11, 12, or 13 or a variant thereof.

The variant is at least 80% identical to amino acid sequence SEQ ID NO: 5, 7, 8, 9, 10, 11, 12 or SEQ ID NO: 13.

In one further embodiment, the nucleic acid comprises a sequence of SEQ ID NO: 6, or a variant thereof.

In a further embodiment, the amino acid sequence of the encoded mutant hdCK is at least 80% identical to sequence SEQ ID NO: 1 of the wild-type hdCK, preferably at least 85% identical to sequence SEQ ID NO: 1, more preferably at least 90% identical to sequence SEQ ID NO: 1, still more preferably at least 95%, at least 96%, at least 97%, at least 98% or at least 99% identical to sequence SEQ ID NO: 1.

In a further embodiment, the variant is at least 80% identical to amino acid sequence SEQ ID NO: 5, preferably provided that said sequence contains the at least four mutations at the amino acid positions S169, E171, E247 and L249.

The inventors have shown that the mutated hdCK of the invention increases the phosphorylation of nucleoside analogue in comparison to a reference hdCK.

Accordingly, in one aspect of the invention the encoded mutated hdCK increases the phosphorylation of a nucleoside analogue.

Terms such as "decreased", "higher", "less", "smaller", "increased", "lower" or "less" or such alike denote quantitative differences between two states and refer to at least statistically significant differences between the two states.

"Increased phosphorylation" in context of the invention refers to the phosphorylation of a nucleoside analogue that has been qualitatively and/or quantitatively evaluated and which is increased in comparison to the phosphorylation of the same nucleoside analogue in the same experimental conditions by a reference hdCK.

In one embodiment, the reference hdCK is the full-length wild-type hdCK of amino acid sequence SEQ ID NO: 1 or the so-called mutant "G12" hdCK of amino acid sequence SEQ ID NO: 3, the reference is preferably the wild-type hdCK of sequence SEQ ID NO: 1.

Methods to qualitatively and/or quantitatively evaluate phosphorylation of a compound, such as a nucleoside compound, are known to the skilled in the art. In one example, the efficiency of phosphorylation of a substrate such as the natural substrate deoxycytidine, or the prodrugs gemcitabine and AraC, is measured for example with the purified wild-type hdCK and the mutant "M36" in typically a NADH-based assay as described in (Agarwal KC. et al. 1978 Methods Enzymol 51: 483-490). Accordingly, the enzymes are typically assayed at, for example, room temperature at a concentration of 0.45 µM for dC, 0.3 µM for Gemcitabine, and 0.3 for AraC. dC is typically used at concentrations of 5 to 50 µM, Gemcitabine at concentrations of 20 µM to 1 mM, and araC is typically used at concentrations of 20 µM to 0.5 mM. ATP is used typically at 4 mM.

In some embodiments, the mutations present in the hdCK have an impact on substrate specificity resulting in impaired phosphorylation activity of the natural substrate deoxycytidine. Accordingly, in some embodiments the encoded mutated hdCK does not significantly phosphorylate deoxycytidine.

In a further embodiment, the encoded mutated hdCK increases the sensitivity of eukaryotic cells to a nucleoside analogue by at least a factor 2.5 when compared to a reference hdCK in a eukaryotic cell.

Methods to measure sensitivity of a cell, such as a eukaryotic cell are known to the skilled in the art. In one example, cells were transduced with a vector comprising for example the mutant "M36" or the wild-type hdCK. Therefore, 1×10⁶ cells were contacted with 1 ml of 1:5 diluted viral vectors rescued from the isolated clones. Transduced cells were selected, for example, by adding puromycin 24h after transduction using 0.6 µg/ml to HEK-293T cells, 0.6 µg/ml to HEK-293T-CD4⁺ cells, 0.5 µg/ml to Messa10K cells. The transduced cells were then for example seeded at 5,000 cells per well in a 96-well plate and grown overnight at 37°C. Typically, two rows were used for each population. 12h after seeding, increasing concentrations of for example gemcitabine (0-400 nM or 0-100mM), or araC (0-10 mM) were added to each well and left in culture for further 72h. Cell viability was measured by MTT test (CellTiter 96 Non-Radioactive Cell Proliferation Assay, Promega) and the number of living cells in each well was evaluated by measuring the OD at 570 nm. For each population, the fraction of living cells was calculated as OD570 concentration X Gem/OD570 concentration 0 Gem, to estimate the sensitivity of the population to the prodrug.

In one embodiment, the encoded mutated hdCK has an IC50 in Messa10K cells to gemcitabine that is less than 30nM.

"IC50" or "IC₅₀" refers to the half maximal inhibitory concentration and is a measure of the effectiveness of a substance in inhibiting a specific biological or biochemical function. In particular, the IC50 value indicates how much of a particular drug or other substance is needed to inhibit a given biological process (or component of a process, i.e. an enzyme, cell, cell receptor or microorganism) by half. According to the FDA, IC₅₀ represents the concentration of a drug that is required to induce a response halfway between the baseline and maximum after a specified exposure time.

It will be understood by a skilled person that numerous different nucleic acids can encode the same polypeptide as a result of the degeneracy of the genetic code. In addition, it is to be understood that skilled persons may, using routine techniques, make nucleotide substitutions that do not affect the polypeptide sequence encoded by the nucleic acids of the invention to reflect the codon usage of any particular host organism in which the polypeptides are to be expressed. The nucleic acids may be modified by any method available in the art. Such modifications may be carried out in order to enhance the *in vivo* activity or life span of the nucleic acids of the invention.

Nucleic acids may be produced recombinantly, synthetically, or by any means available to those of skill in the art. They may also be cloned by standard techniques such as PCR (polymerase chain reaction).

In one embodiment, the nucleic acid of the invention contains only a coding region for the mutant hdCK of the invention. However, in some embodiments, the nucleic acid further comprises, in operable linkage, a portion of nucleic acid that allows for efficient translation of the coding sequence in the target cell. Accordingly, in one embodiment, the nucleic acid (when in a DNA form) further comprises a promoter in operable linkage which allows for the transcription of the coding region and the portion of nucleic acid that allows for efficient translation of the coding region in the target cell. Those elements are further defined in the section « vector » herein below.

### Vectors

In one embodiment, nucleic acids of the invention are incorporated into a vector.

As it is well known in the art, a vector is a tool that allows or facilitates the transfer of an entity from one environment to another.

In context of the present invention "vector" refers to vectors used in recombinant DNA techniques allowing entities, such as a nucleic acids, to be transferred into a host and/or a target cell for the purpose of replicating the vectors comprising the nucleotide sequences used in the invention and/or expressing the proteins encoded by the nucleic acid of the invention. Examples of vectors used in recombinant DNA techniques include but are not limited to plasmids, chromosomes, artificial chromosomes or viruses.

Accordingly, in one embodiment the vector is also a so-called "delivery system". The vector is preferably an isolated vector.

In a further embodiment the vector is an expression vector.

An "expression vector" herein refers to a vector as defined above designed for protein expression in cells. The vector is engineered to contain regulatory sequences that act as enhancer and promoter regions and lead to efficient transcription of the gene carried on the expression vector. Those regulatory sequences may as well be called "expression signal" or "control sequences". Accordingly, in one embodiment, a nucleic acid in a vector is operably linked to a control sequence that is capable of providing for the expression of the coding sequence by the host cell.

The term "operably linked" means that the components described are in a relationship permitting them to function in their intended manner. A regulatory sequence "operably linked" to a coding sequence is ligated in such a way that expression of the coding sequence is achieved under conditions compatible with the control sequences.

In one embodiment, the vector preferably comprises a mutant hdCK expression cassette, *i.e.* a nucleic acid encoding a mutant hdCK of the invention operably linked to at least one expression signal allowing its expression.

The at least one expression signal is particularly selected among a promoter, a terminator, an enhancer and their combinations, preferably among a promoter, a terminator.Suitable promoters, terminators and enhancers are well-known by the skilled person and may be selected to be compatible with the host cell for which the expression vector is designed to be used in.

The control sequences may be modified, for example by the addition of further transcriptional regulatory elements to make the level of transcription directed by the control sequences more responsive to transcriptional modulators.

The term "promoter" is well-known in the art and encompasses nucleic acid regions ranging in size and complexity from minimal promoters to promoters including upstream elements and enhancers. The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of a-actin, b-actin, tubulin, human elongation factor1-alpha (EF1-alpha)) or, alternatively, a tissue-specific manner (such as promoters of the genes for pyruvate kinase). Viral promoters may also be used, for example the moloney murine leukaemia virus long terminal repeat (MMLV LTR) promoter, the rous sarcoma virus (RSV) LTR promoter or the human cytomegalovirus (CMV) IE promoter.

In some embodiments, the promoters are inducible so that the levels of expression of the heterologous gene can be regulated during the life-time of the cell. Inducible means that the levels of expression obtained using the promoter can be regulated.

Chimeric promoters may also be used comprising sequence elements from two or more different promoters described above.

In one embodiment, the vectors used in the present invention further comprise an origin of replication.

In a further embodiment, the vectors further contain one or more selectable marker genes, and/or a traceable marker such as GFP. In one embodiment, said selectable marker gene and/or traceable marker gene may be a reporter gene. Reporter genes are further defined in the section "Therapeutic methods and uses".

The vectors of the invention may be used, for example, to transfect or transform a host cell.

For example, the promoter is the human elongation factor1-alpha (EF1-alpha) promoter.

For example, the terminator is the polyA signal in the 5' LTR.

In one embodiment, the vectors of the invention do not comprise an enhancer.

In one embodiment, a mutant hdCK expression cassette comprises the human elongation factor1-alpha (EF1-alpha) promoter, a nucleic acid encoding a human mutant dCK.

In a further embodiment, a mutant hdCK expression cassette comprises the human elongation factor1-alpha (EF1-alpha) promoter, a nucleic acid encoding a human mutant dCK followed directly by a human gene promoter that drives the expression of a reporter gene and a reporter gene. Preferably, said mutant hdCK expression cassette does not contain a termination signal before the human gene promoter that drives the expression of a reporter gene.

The vector comprising a nucleic acid encoding a mutant hdCK of the invention is preferably a non-viral vector or a viral vector.

A non-viral vector may be selected in the group consisting of a plasmid, a liposomal nucleic acid complex and a carrier-associated nucleic acid.

By "plasmid", it is herein meant a double-stranded circular DNA. The plasmid may include a marker gene enabling to select the cells comprising said plasmid, an origin of replication to allow the cell to replicate the plasmid and/or a multiple cloning site allowing the insertion of a DNA fragment, in particular the nucleic acid encoding a mutant hdCK of the invention.

Non-limitative examples of carrier-associated nucleic acids are polymer-carried DNA and cationic lipids.

The liposomes used in liposomal DNA complexes are well-known in the art. Said liposomes may be cationic, anionic or neutral liposomes.

Cationic lipids are also known in the art and are commonly used for gene delivery. Such lipids include Lipofectin Tm also known as DOTMA (N- [I- (2, 3-dioleyloxy) propyls N, N, N-trimethylammonium chloride), DOTAP (1, 2-bis (oleyloxy)-3 (trimethylammonio) propane), DDAB (dimethyldioctadecyl- ammonium bromide), DOGS (dioctadecylamidologlycyl spermine) and cholesterol derivatives such as DC-Chol (3 beta-(N- (N', N'- dimethyl aminomethane)-carbamoyl) cholesterol). Cationic lipids for gene delivery are preferably used in association with a neutral lipid such as DOPE (dioleyl phosphatidylethanolamine).

In a preferred embodiment, the vector comprising a nucleic acid encoding a mutant hdCK is a viral vector.

By "viral vector", it is herein meant a recombinant viral vector.

The viral vector is preferably selected in the group consisting of a retrovirus vector, a lentivirus vector, Herpes simplex viral vector, an adenovirus vector and an adeno-associated virus vector, preferably a lentivirus vector.

A "retrovirus vector" herein refers to a genetically engineered a retrovirus based vector that cannot replicate and produce progeny infectious virus particles once the virus has entered the target cell. A retrovirus vector genome consists in a single-stranded positive-sense RNA that encodes a transcriptase enabling to generate double-stranded DNA.

There are many retroviruses that are widely used for delivery of genes both in tissue culture conditions and in living organisms. Examples include and are not limited to murine leukemia virus (MLV), human immunodeficiency virus (HIV-1), equine infectious anaemia virus (EIAV), mouse mammary tumour virus (MMTV), rous sarcoma virus (RSV), fujinami sarcoma virus (FuSV), moloney murine leukemia virus (Mo-MLV), FBR murine osteosarcoma virus (FBR MSV), moloney murine sarcoma virus (Mo-MSV), abelson murine leukemia virus (A-MLV), avian myelocytomatosis virus-29 (MC29), and avian erythroblastosis virus (AEV) and all other retroviridiae including lentiviruses. A detailed list of retroviruses may be found in Coffin et al., 1997, "retroviruses", Cold Spring Harbour Laboratory Press Eds: JM Coffin, SM Hughes, HE Var us pp 758-763.

The basic structure of a retrovirus genome is a 5' LTR and a 3' LTR, between or within which are located a packaging signal to enable the genome to be packaged, a primer binding site, integration sites to enable integration into a host cell genome and gag, pol and env genes encoding the structural and enzymatic components - these are polypeptides required for the assembly of viral particles. More complex retroviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for pro viral integration, and transcription. LTRs also serve as enhancer-promoter sequences and can control the expression of the viral genes. Encapsidation of the retroviral RNAs occurs by virtue of a psi sequence located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In one embodiment, the LTRs are replaced with self-inactivating LTRs.

In a defective retroviral vector genome gag, pol and env may be absent or not functional. The R regions at both ends of the RNA are repeated sequences. U5 and

U3 represent unique sequences at the 5' and 3' ends of the RNA genome respectively.

More preferably, the viral vector is a targeted vector that is it has a tissue tropism which is altered compared to the native virus, so that the vector is targeted to particular cells. This may be achieved by modifying the retroviral Env protein. Preferably the envelope protein is a non-toxic envelope or an envelope which may be produced in non-toxic amounts within the primary target cell, such as for example a MMLV amphotropic envelope or a modified amphotropic envelope.

Preferably the envelope is one which allows transduction of human cells. Examples of suitable env genes include, but are not limited to, VSV-G, a MLV amphotropic env such as the 4070 A env, the RD114 feline leukaemia virus env or haemagglutinin (HA) from an influenza virus. The Env protein may be one which is capable of binding to a receptor on a limited number of human cell types and may be an engineered envelope containing targeting moieties. The env and gag-pol coding sequences are transcribed from a promoter and optionally an enhancer active in the chosen packaging cell line and the transcription unit is terminated by a polyadenylation signal. For example, if the packaging cell is a human cell, a suitable promoter-enhancer combination is that from the human cytomegalovirus major immediate early (hCMV-MIE) gene and a polyadenylation signal from SV40 virus may be used. Other suitable promoters and polyadenylation signals are known in the art.

In one embodiment, the retrovirus vector is a murine leukemia virus (MLV) vector. Retroviral vectors derived from the amphotropic Moloney murine leukemia virus (MLV-A) are commonly used in clinical protocols worldwide.

The "lentivirus vector" is a single-stranded RNA that integrates in the genome of the host, thereby allowing long term expression of the nucleic acid. The lentivirus is a genus of viruses of the retroviridae family, characterized by a long incubation period. A distinction between the lentivirus family and other types of retroviruses is that lentiviruses have the capability to infect both dividing and non-dividing cells (Lewis, 1992; Lewis and Emerman, 1994). A detailed list of lentiviruses may be found in Coffin et al. ("Retroviruses" 1997 Cold Spring Harbour Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human acquired-immunodeficiency syndrome (AIDS), and the simian immunodeficiency virus (SIV). The non-primate lentiviral group includes the prototype "slow virus" visna maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BTV). In one embodiment the lentiviral vector is a HIV-based VSV-pseudotyped defective vector.

"Herpes simplex virus" (HSV) is an enveloped, double-stranded (ds) DNA virus. The mature virion consists of different components: an external envelope containing about 13 glycoproteins involved in different functions, among which the first steps of binding and entry into the host cell; an amorphous layer known as the tegument, containing some 20 different proteins with structural and regulatory roles; and an icosadeltahedral capsid containing a toroidal dsDNA. In one embodiment, the Herpes simplex virus vector is in particular a HSV-1 vector. Different types of HSV-1 vectors a known to the skilled in the art, such as amplicons, replication-defective and replication-competent vectors.

The "amplicons" are plasmid-derived vectors engineered to contain both the origin of HSV DNA replication *(ori)* and HSV cleavage-packaging recognition sequences (pac). When amplicons are transfected into mammalian cells with HSV helper functions, they are replicated, form head-to-tail linked concatamers and are then packaged into viral particles. There are two major methods currently used for producing amplicon particles, one based on infection with defective helper HSVs and the other based on transfection of HSV-1 genes, such as a set of pac-deleted overlapping cosmids or a pac-deleted and ICP27-deleted BAC-HSV-1.

"Replication-defective vectors" are made of mutant viruses with deletions in one or more genes essential for the lytic cycle, whereas "replication-competent vectors" are composed of attenuated viruses where genes that are not essential for replication in cultured cells *in vitro* are either mutated or deleted (Roberto Manservigi et al. 2010, "HSV Recombinant Vectors for Gene Therapy", Open Virol J. 2010; 4: 123-156).

The "adenovirus" is a double-stranded, linear DNA virus that does not go through an RNA intermediate. There are over 50 different human serotypes of adenovirus divided into 6 subgroups based on the genetic sequence homology. The natural target of adenovirus is the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses are non-enveloped, regular icosohedrons. A typical adenovirus comprises a 140nm encapsidated DNA virus. The icosahedral symmetry of the virus is composed of 152 capsomeres: 240 hexons and 12 pentons. The core of the particle contains the 36kb linear duplex DNA which is covalently associated at the 5' ends with the Terminal Protein (TP) which acts as a primer for DNA replication. The DNA has inverted terminal repeats (ITR) and the length of these varies with the serotype.

The adenovirus is a double stranded DNA non-enveloped virus that is capable of *in vivo* and *in vitro* transduction of a broad range of cell types of human and non-human origin. These cells include respiratory airway epithelial cells, hepatocytes, muscle cells, cardiac myocytes, synoviocytes, primary mammary epithelial cells and post-mitotically terminally differentiated cells such as neurons.

Adenoviral vectors are also capable of transducing non dividing cells. This is very important for diseases, such as cystic fibrosis, in which the affected cells in the lung epithelium have a slow turnover rate. In fact, several trials are underway utilizing adenovirus-mediated transfer of cystic fibrosis transporter (CFTR) into the lungs of afflicted adult cystic fibrosis patients.

Adenoviruses have been used as vectors for gene therapy and for expression of heterologous genes. The large (36 kilobase) genome can accommodate up to 8kb of foreign insert DNA and is able to replicate efficiently in complementing cell lines to produce very high titres of up to 10¹² . Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, it functions episomally (independently from the host genome) as a linear genome in the host nucleus.

An "adeno-associated virus vector", also called AAV is a single-stranded, either positive- or negative-sensed DNA, virus, with an apparent lack of pathogenicity. It infects dividing and non-dividing cells and has the ability to stably integrate into the host cell genome at a specific site (designated AAVS1) in the human chromosome 19. The AAV genome integrates most frequently into the mentioned site, while random incorporations into the genome take place with a negligible frequency. The genome comprises inverted terminal repeats (ITRs) at both ends of the DNA strand, and two open reading frames (ORFs): rep and cap. The former is composed of four overlapping genes encoding Rep proteins required for the AAV life cycle, and the latter contains overlapping nucleotide sequences of capsid proteins: VP1, VP2 and VP3, which interact together to form a capsid of an icosahedral symmetry. Removal of the *rep* and cap from the DNA of the vector eliminates the above-mentioned integrative capacity.

In one embodiment, an adeno-associated virus vector is selected from the group consisting of an AAV1 vector, AAV2 vector, AAV6 vector, AAV8 vector, AAV9 vector and a hybrid AAV vector.

A "hybrid AAV vector" is a vector comprising the rep gene of one AAV vector and the cap gene of another AAV vector.

Non-limitative examples of hybrid AAV vector are an AAV1/2 vector, i.e. a vector comprising AAV1 rep gene and AAV2 cap gene, or an AAV2/9 vector, i.e. a vector comprising with AAV2 rep gene and AAV9 cap gene.

An example of AAV2/9 vector is pZac2.1 (for example from Penn Vector Core, Upenn).

### Host cells

Vectors and nucleic acids of the invention may be introduced into host cells for the purpose of replicating the vectors/nucleic acids and/or expressing the mutant hdCK encoded by the nucleic acid of the invention.

In one embodiment, the host cell may be a bacterial cell or a eukaryotic cell.

In one embodiment, said host cell is a prokaryotic cell, wherein said prokaryotic cells may be selected from the group including but not limited to *E. coli, Bacillus subtilis, Salmonella typhimurium* and species within the genera *Pseudomonas, Streptomyces,* and *Staphylococcus.*

In a further embodiment, said host cell is an eukaryotic cell, wherein said eukaryotic cell may be selected from the group including but not limited to a human cell, a dog cell, a monkey cell, a rat cell, a mouse cell, a pig cell.

In a further embodiment the eukaryotic cell is selected from the group consisting of human stem cells (both embryonic and of later ontogeny), and human precursor cells, such as human neural stem or precursor cells, human hematopoietic stem or precursor cells, cells of the immune system such as T-cells, bone marrow cells such as adherent and non-adherent bone marrow cells, peripheral blood mononuclear cells such as dendritic cells, human hepatocyte progenitors, and mesenchymal stem cells or cancer cells.

In a further embodiment, the host cell may be a cell for packaging and propagating a virus, such as retroviral packaging cell lines which are well known in the art.

In another embodiment, the host cell may be a cell in a subject (whether human or animal) which is desired to be destroyed. Accordingly, in one preferred embodiment, the host cell is a cancer cell. The nucleic acid and vector of the present invention are useful to target to said cells to be destroyed. Accordingly, cells expressing the mutant hdCK may be contacted with an agent which is substantially non-toxic and which is converted to a toxic form by the mutant hdCK.

### Polypeptides

In a further aspect of the invention vectors or nucleic acids of the invention may be transformed or transfected into a suitable host to provide for expression of the mutated hdCK product. This process may comprise *in vitro* culturing a host cell transformed with an expression vector, as described above, under conditions to provide for expression by the vector of a coding sequence encoding the protein, and optionally recovering the expressed protein.

Accordingly, the invention refers to a process for producing a mutant human deoxycytidine kinase (hdCK) comprising *in vitro* culturing a host cell, as described above in the section "host cell" and recovering the expressed mutant hdCK from the cultured host cells and/or culture medium.

The mutant dCK of the invention is herein also referred to as "polypeptide" or "peptide".

As used herein, the terms "polypeptide" and "peptide" refer to a polymer in which the monomers are amino acids and are joined together through peptide or disulfide bonds. The terms subunit and domain may also refer to polypeptides and peptides having biological function.

In one embodiment, the human mutant deoxycytidine kinase is a protein.

As used herein, the term "protein" includes single-chain polypeptide molecules as well as multiple-polypeptide complexes where individual constituent polypeptides are linked by covalent or non-covalent means.

Accordingly the invention further refers to an isolated mutant human deoxycytidine kinase (hdCK) comprising a polypeptide sequence which differs from wild-type hdCK of sequence SEQ ID NO: 1 by at least one mutation, wherein the at least one mutation is at the amino acid position S169 in comparison to the amino acid sequence SEQ ID NO: 1.

In a further embodiment, the invention refers to an isolated mutant human deoxycytidine kinase (hdCK) comprising a polypeptide sequence which differs from wild-type hdCK of sequence SEQ ID NO: 1 by at least one mutation, wherein the at least one further mutation is selected at the position E171, E247 or L249, in comparison to the amino acid sequence SEQ ID NO: 1.

The at least one further mutation is as defined above in the section "polynucleotide".

Accordingly, the invention further refers to an isolated mutant dCK comprising a polypeptide sequence which differs from wild-type hdCK of sequence SEQ ID NO: 1 by at least two mutations, wherein, the at least two mutations are
a) at the amino acid positions S169 and E171, or
b) at the amino acid positions S169 and L249, or
c) at the amino acid positions S169 and L249.

In a further embodiment, the polypeptide sequence differs from wild-type hdCK of sequence SEQ ID NO: 1 by at least three mutations, wherein the at least three mutations are
a) at the amino acid positions S169, E171 and E247, or
b) at the amino acid positions S169, E171 and E249, or
c) at the amino acid positions S169, E247 and E249.

In a further embodiment, the polypeptide sequence differs from wild-type hdCK of sequence SEQ ID NO: 1 by at least four mutations, wherein the at least four mutations are at the amino acid positions S169, E171, E247 and E249.

In one embodiment, the mutation at position S169 is selected from the substitutions S169N, S169T, and S169Q, preferably S169N.

In one embodiment, the mutation at position E171 is selected from the substitutions E171 K, and E171 R, preferably E171 K.

In one embodiment, the mutation at position E247 is selected from the substitutions E247K, and E247R, preferably E247K.

In one embodiment, the mutation at position L249 is selected from the substitutions L249M, L249F, L249Y, L249I and L249W, preferably L249M.

In a further embodiment, said mutant hdCK is the so called mutant "M36" and comprises a polypeptide sequence which differs from wild-type hdCK of sequence SEQ ID NO: 1 by at least four mutations, wherein the at least four mutations are S169N, E171 K, E247K and L249M.

In one embodiment, the amino acid sequence of the mutant hdCK comprises the amino acid sequence SEQ ID NO: 5, 7, 8, 9, 10, 11, 12, or 13, or a variant thereof.

In one embodiment, the variant is at least 80% identical to amino acid sequence SEQ ID NO: 5, 7, 8, 9, 10, 11, 12 or 13.

In a further embodiment, the amino acid sequence of the mutant dCK is at least 80 % identical to sequence SEQ ID NO: 1 of the human wild-type deoxycytidine kinase, preferably at least 85% identical to sequence SEQ ID NO: 1, more preferably at least 90% identical to sequence SEQ ID NO: 1, still more preferably at least 95%, at least 96%, at least 97%, at least 98% or at least 99% identical to sequence SEQ ID NO: 1.

In a further embodiment, the amino acid sequence of the mutant dCK is at least 80 % identical to sequence SEQ ID NO: 5, preferably provided that said sequence contains the at least four mutations at the amino acid positions S169, E171, E247 and L249.

### Pharmaceutical compositions

The isolated nucleic acid, the expression vector, the host cell or the isolated mutant hdCK of the invention may be combined with pharmaceutically acceptable excipients, and optionally sustained-release matrices, such as biodegradable polymers, to form therapeutic compositions.

Thus, another object of the invention relates to a pharmaceutical composition comprising an isolated nucleic acid, an expression vector, a host cell, or an isolated mutant hdCK of the invention and a pharmaceutically acceptable carrier.

In one embodiment the pharmaceutical composition comprises an isolated nucleic acid, an expression vector, or an isolated mutant hdCK of the invention and a pharmaceutically acceptable carrier.

The isolated nucleic acid, the expression vector, the host cell, or the isolated mutant hdCK are as defined above in the sections above "polynucleotides", "vector", "host cell" and "polypeptides".

The invention also relates to an isolated nucleic acid, an expression vector, a host cell, or an isolated mutant hdCK according to the invention, for use as a medicament. In one embodiment, the invention relates to an isolated nucleic acid, an expression vector, or an isolated mutant hdCK according to the invention, for use as a medicament.

The invention also relates to a pharmaceutical composition of the invention for use as a medicament.

In one embodiment, the invention refers to an isolated nucleic acid, an expression vector, a host cell or an isolated mutant hdCK according to the invention or a pharmaceutical composition thereof in combination with a nucleoside analogue for use as a medicament, preferably the invention refers to an isolated nucleic acid, an expression vector, or an isolated mutant hdCK according to the invention or a pharmaceutical composition thereof in combination with a nucleoside analogue for use as a medicament

In a further embodiment, the invention refers to a nucleoside analogue in combination with an isolated nucleic acid, an expression vector, a host cell or an isolated mutant hdCK according to the invention or a pharmaceutical composition thereof for use as a medicament, preferably the invention refers to a nucleoside analogue in combination with an isolated nucleic acid, an expression vector, or an isolated mutant hdCK according to the invention or a pharmaceutical composition thereof for use as a medicament.

The terms "pharmaceutical composition" or "therapeutic composition" as used herein refer to a compound or composition capable of inducing a desired therapeutic effect when properly administered to a patient.

Such therapeutic or pharmaceutical compositions may comprise a therapeutically effective amount of an isolated nucleic acid, an expression vector, a host cell or an isolated mutant hdCK of the invention, in admixture with a pharmaceutically or physiologically acceptable formulation agent selected for suitability with the mode of administration.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Pharmaceutically-acceptable carriers can be prepared by any method known by those skilled in the art.

As used herein, "pharmaceutically-acceptable carriers" may comprise excipients and auxiliaries, which facilitate processing of the active compounds into preparations which can be used pharmaceutically. Suitable pharmaceutically acceptable carriers are described for example in Remington's Pharmaceutical Sciences (Mack Publishing Company, Easton, USA, 1985), which is a standard reference text in this field. Pharmaceutically acceptable carriers can be routinely selected in accordance with the mode of administration, solubility and stability of nucleic acids, vectors, host cells or isolated mutant hdCKs of the invention. For example, formulations for intravenous administration may include sterile aqueous solutions which may also contain buffers, diluents and other suitable additives. The use of biomaterials and other polymers for drug delivery, as well the different techniques and models to validate a specific mode of administration, are disclosed in literature.

Pharmaceutically-acceptable carriers thus includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, dextrose, glycerol, ethanol, and the like, as well as combination thereof. In many cases, it will be preferable to include isotonic agents, such as sugars, polyalcohols, or sodium chloride in the composition and formulation may also contain an antioxidant such as tryptamine and a stabilizing agent such as Tween 20.

The form of the pharmaceutical compositions, the route of administration, the dosage and the regimen naturally depend upon the condition to be treated, the severity of the illness, the age, weight, and gender of the patient, etc.

The pharmaceutical compositions of the invention can be formulated for a topical, oral, parenteral, intranasal, intravenous, intramuscular, subcutaneous or intraocular administration and the like.

In particular, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the isolated nucleic acid, the expression vector, the host cell or the isolated mutant hdCK of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The nucleic acid or vector according to the invention is preferably formulated as liquid (e.g., solutions, suspensions).

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An isolated nucleic acid, an expression vector, a host cell or an isolated mutant hdCK of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, glycine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The amount of, for example, nucleic acid or vector to be used in a pharmaceutical composition depends, for example, on the strength of the promoter used in the DNA construct, the immunogenicity, the condition of the mammal intended for administration (e. g., weight, age, sex and health, concurrent treatment, if any, frequency of treatment), the mode of administration and the type of formulation.

For example, the pharmaceutical composition may comprise from 1 µg to 8 mg, preferably from 1 µg to 1 mg, preferably from 10 µg to 800 µg, more preferably from 25 µg to 250 µg, of the nucleic acid or vector, in particular for administration to human adults and/or when using a non-viral vector.

The administration may be achieved in a single dose or several doses.

Once the pharmaceutical composition has been formulated, it can be stored in sterile vials as a solution, suspension, gel, emulsion, solid, or as a dehydrated or lyophilized powder. Such formulations can be stored either in a ready-to-use form or in a form (e.g., lyophilized) requiring reconstitution prior to administration.

In a preferred embodiment, the pharmaceutical compositions are presented in unit dosage forms to facilitate accurate dosing. The term "unit dosage forms" refers to physically discrete units suitable as unitary dosages for human subjects and other mammals, each unit containing a pre-determined quantity of active material calculated to produce the desired therapeutic effect, in association with a suitable pharmaceutical excipient. Typical unit dosage forms include pre-filled, pre-measured ampoules or syringes of the liquid compositions. In such compositions, the nucleic acid or vector is usually a minor component, with the remainder being various vehicles or carriers and processing aids helpful for forming the desired dosing form.

In one embodiment, guidance to the dosage of an isolated nucleic acid, an expression vector, a host cell or an isolated mutant hdCK of the invention and nucleoside analogs can be found in the numerous publications describing clinical trials with HSV-TK1 suicide gene therapy. Thymidine kinases, in particular human HSV-TK1 have been used extensively as suicide gene therapy for the treatment of various types of cancer in combination with various nucleoside analogues. E.g. [Klatzmann D, Valery CA, Bensimon G, Marro B, Boyer O, Mokhtari K, Diquet B, Salzmann JL, Philippon J. A phase I/II study of herpes simplex virus type 1 thymidine kinase "suicide" gene therapy for recurrent glioblastoma. Study Group on Gene Therapy for Glioblastoma. Hum Gene Ther. 1998 Nov 20;9(17):2595-604.]; [Klatzmann D, Cherin P, Bensimon G, Boyer O, Coutellier A, Charlotte F, Boccaccio C, Salzmann JL, Herson S. A phase I/II dose-escalation study of herpes simplex virus type 1 thymidine kinase "suicide" gene therapy for metastatic melanoma. Study Group on Gene Therapy of Metastatic Melanoma. Hum Gene Ther. 1998 Nov 20;9(17):2585-94.]; [Freytag SO, Strieker H, Pegg J, Paielli D, Pradhan DG, Peabody J, DePeralta-Venturina M, Xia X, Brown S, Lu M, Kim JH. Phase I study of replication-competent adenovirus-mediated double-suicide gene therapy in combination with conventional-dose three-dimensional conformal radiation therapy for the treatment of newly diagnosed, intermediate- to high-risk prostate cancer. Cancer Res. 2003 Nov 1;63(21):7497-506.]; [Freytag SO, Khil M, Strieker H, Peabody J, Menon M, DePeralta-Venturina M, Nafziger D, Pegg J, Paielli D, Brown S, Barton K, Lu M, Aguilar-Cordova E, Kim JH. Phase I study of replication-competent adenovirus-mediated double suicide gene therapy for the treatment of locally recurrent prostate cancer. Cancer Res. 2002 Sep 1 ;62(17):4968-76.]; [Sung MW, Yeh HC, Thung SN, Schwartz ME, Mandeli JP, Chen SH, Woo SL. Intratumoral adenovirus-mediated suicide gene transfer for hepatic metastases from colorectal adenocarcinoma: results of a phase I clinical trial. Mol Ther. 2001 Sep;4(3): 182-91.]; [Packer RJ, Raffel C, Villablanca JG, Tonn JC, Burdach SE, Burger K, LaFond D, McComb JG, Cogen PH, Vezina G, Kapcala LP. Treatment of progressive or recurrent pediatric malignant supratentorial brain tumors with herpes simplex virus thymidine kinase gene vector-producer cells followed by intravenous ganciclovir administration. J Neurosurg. 2000 Feb;92(2):249-54.].

HSV-TK has been used for treating the following types of cancer, which are amenable to suicide gene therapy according to the present invention. Bladder cancer, Sutton et al. 1997, Urology, 49:173-180; Neuroblastoma, Bi, X and Zhang, J-Z. Pediadtr. Surg. Int., 19:400-405, 2003; Glioblastoma, Germano I. M et al. J. Neurooncol., 65:279-289, 2003; Esophageal cancer, Matsubara, H. and Ochiai, Nippon Rinsho. 2000 Sep;58(9):1935-43.; Tongue cancer, Wang, J. H. et al. Chin J. Dent. Res. 2000, Dec. 3(4): 44-48; Hepatocellular carcinoma, Gerolami, R. et al. J. Hepatol. 291-297, 2004; Lung cancer, Kurdow, R. et al. Ann. Thorac. Surg. 2002 Mar; 73(3):905- 910; Malignant melanoma, Yamamoto, S. et al. Cancer Gene Therapy, 10:179-186, 2003; Ovarian cancer, Barnes, M.N. and Pustilnik, T.B. Curr. Opin. Obstet Gynecol., 13:47-51 , 2001 ; Prostate cancer. Kubo, H. et al. Human Gene Therapy., 14:227-241, 2003; Renal cell carcinoma, Pulkkanen, K.J. Cancer Gene Therapy, 9:908-916, 2002.

Adaptation of the dosages described in the above identified publications to the hdCKs described in the present application are within the capabilities of the person skilled in the art.

### Kits

The invention further provides kits comprising a pharmaceutical composition comprising an isolated nucleic acid, an expression vector, a host cell or an isolated mutant hdCK according to the invention and a nucleoside analogue, preferably the kit comprises a pharmaceutical composition comprising an isolated nucleic acid, an expression vector, or an isolated mutant hdCK according to the invention and a nucleoside analogue.

Nucleoside analogues have been defined above in the section "definition".

In one embodiment, the kit further comprises instructions regarding the mode of administration. These instructions may e.g. indicate the medical indication, the route of administration, the dosage and/or the group of patients to be treated.

### Therapeutic methods and uses

The inventors have shown that the mutated hdCK "M36" increases the sensitivity of M36-expressing cells with respect to control cells expressing an exogenous copy of, for example, the wild-type hdCK gene. The increased sensitivity is demonstrated by a decrease in the IC50 of approximately 10,000 times which was observed for cells containing M36 with respect to cells containing the wild-type hdCK as exogenous gene. The mutated hdCK of the invention therefore increases sensitivity of the cells to the exposure of anticancer chemicals.

Accordingly, in one embodiment of the invention, the isolated nucleic acid, expression vector, host cell or isolated mutant hdCK of the invention, or pharmaceutical composition thereof, are for use as a medicament. Preferably, the isolated nucleic acid, expression vector or isolated mutant hdCK of the invention or pharmaceutical composition thereof are for use as a medicament for treating or preventing a disease or disorder in a subject.

The invention further refers to a method of inhibiting a pathogenic agent in a subject, which method comprises administering to said subject a nucleic acid or an expression vector of the invention.

In one embodiment, said nucleic acid or said expression vector is administered *in vivo.*

In a further embodiment, said nucleic acid or said expression vector is administered *ex vivo.*

In a further embodiment, said nucleic acid or said expression vector is administered *in vitro.*

In one embodiment, said pathogenic agent is a virus or a cancer cell.

In one embodiment the method of inhibiting a pathogenic agent in a subject further comprises the step of administering a nucleoside analogue to said subject.

In one embodiment, said nucleoside analogue is administered *in vivo, ex vivo* or *in vitro, preferably in vivo.*

Nucleoside analogs are as defined in the section "definition" herein above.

The invention further refers to an *in vitro* method of phosphorylating a nucleoside or nucleoside analogue comprising the steps of:
(i) subjecting the nucleoside or nucleoside analogue to the action of the mutant hdCKas described above in the section "polypeptides", and
(ii) recovering the phosphorylated nucleoside or nucleoside analogue.

In one embodiment the invention provides a method of treating or preventing a disease or disorder comprising administering to a subject in need thereof a therapeutically effective amount of the isolated nucleic acid, a vector, an isolated mutant hdCK, or a host cell of the invention or a pharmaceutical composition of the invention as defined above in the section "pharmaceutical composition".

In a particular embodiment, the nucleic acid of the invention is for use as a safety gene in gene therapy or cell therapy.

"Safety gene" herein refers to a nucleotide sequence which can be used to sensitize the cells having incorporated the transgene to the exposure of anticancer chemicals in case the transgenic cells undergo uncontrolled proliferation or induce severe side effects after engraftment. In one particular embodiment, the nucleic acid of the invention is for use as a safety gene in cell therapy. In one embodiment, the safety gene is used in combination with a transgene of interest for example for use in gene therapy.

Accordingly, in one embodiment, cells of a subject are transfected or transduced with a nucleic acid comprising mutant hdCK.

Accordingly, in a further one embodiment, cells of a subject are transfected or transduced with a nucleic acid comprising mutant hdCK and comprising a further transgene.

"Severe side effects" or "severe adverse events" herein refer to, for example, unwanted immune system reaction such as severe cytokine release syndrome, bleeding, coagulation, rashes or organe failure.

"Cell therapy" herein refers to the administration of cellular material to a patient. Cell therapy includes for example, but is not limited to, allogenic cell therapy, human embryonic stem cell therapy, neural stem cell therapy, mesenchymal stem cell therapy and hematopoic stem cell therapy.

A "transgene" herein refers to a nucleic acid sequence that has been transferred from cells of one organism to cells of another by any of the known genetic engineering techniques. The introduction of a transgene into a cell has the potential to change the phenotype of said cell. In particular, a transgene herein refers to a transgene used in gene therapy such as for example p53, endostatin, active activin receptor-like kinase-2 (caAlk2), VEGF/RANKL, PDGF-B, human β-globin, interleukin-2 receptor subunit gamma). In a further embodiment, a transgene further refers to high affinity tumor-targeting T-cell receptors (TCRs) or synthetic chimeric antigen receptors (CARs).

"Chimeric antigen receptors" (CARs) are synthetic transmembrane proteins that comprise single-chain variable fragments (scFv) derived from monoclonal antibodies fused to components of the TCR signaling transduction machienery and are used to redirect autologous T cells with a new specificity for antigens on the surface of cancer cells. Chimeric antigen receptors (CARs) are distinguished into 1^{st}, 2^{nd} and third generation CARs. "First-generation" CARs typically had the intracellular domain from the CD3 ζ-chain. "Second-generation" CARs add intracellular signaling domains from various costimulatory protein receptors (e.g., CD28, 41 BB, ICOS) to the cytoplasmic tail of the CAR to provide additional signals to the T cell. "Third-generation" CARs combine multiple signaling domains, such as CD3z-CD28-41 BB or CD3z-CD28-OX40.

"High affinity tumor-targeting T-cell receptors (TCRs)" are engineered T-cell receptors directed against a specific antigen that is for example present on a cancer cell.

In one embodiment, in order to enable the monitoring of transfected cells, the cells are transfected with a nucleic acid comprising mutant hdCK and a reporter gene, or the cells are transfected or transduced with a nucleic acid comprising mutant hdCK and a nucleic acid comprising a reporter gene.

In one further embodiment, in order to enable the monitoring of transfected cells, the cells are transfected with a nucleic acid comprising mutant hdCK, a further transgene and a reporter gene, or the cells are transfected or transduced with a nucleic acid comprising mutant hdCK and one nucleic acid comprising a further trangene, wherein one of the nucleic acids further comprises a reporter gene.

A "reporter gene" herein refers to a selectable marker gene and/or a traceable marker such as GFP or EGFP or a gene that, upon expression, alters the bio-distribution of a tracer molecule, leading to its local concentration at the site of the reporter gene expression. Reporter genes that, upon expression, alter the bio-distribution of a tracer which are currently under developement are for example enzymes, receptors, and transport proteins and are further explained in the section "Imaging".

Accordingly, the invention also concerns a method of cell therapy in a subject, which method comprises the steps of:
i) transfecting or transducing cells of a subject with a nucleic acid comprising mutant hdCK and a reporter gene, or transfecting or transducing cells of a subject with a nucleic acid comprising mutant hdCK and one nucleic acid comprising a reporter gene; and
ii) monitoring if said cells undergo uncontrolled proliferation in the subject, and
iii) administering the subject with a nucleoside analogue prodrug, if said transfected or tranduced cells undergo uncontrolled proliferation.

Accordingly, the invention also concerns a method of gene therapy in a subject, which method comprises the steps of:
i) transfecting or transducing cells of a subject with a nucleic acid comprising mutant hdCK, a further transgene and a reporter gene, or transfecting or transducing cells of a subject with a nucleic acid comprising mutant hdCK and one nucleic acid comprising mutant hdCK, wherein one of the nucleic acids further comprises a reporter gene ; and
ii) monitoring if said cells undergo uncontrolled proliferation in the subject, and iii) administering the subject with a nucleoside analogue prodrug, if said transfected or tranduced cells undergo uncontrolled proliferation.

In one embodiment, the reporter gene encodes an enzyme, receptor, or transport protein.

Different methods are known to the skilled in the art to monitor cells that were transfected or transduced with a transgene and are further explained herein below in the section "Imaging".

"Uncontrolled proliferation" herein refers to a proliferation of transduced or transfected cells which can be of prejudice for the subject and is not desired.

In one embodiment, cells are transfected or transduced in step i) of the method of gene therapy *in vivo, ex vivo or in vitro.*

In analogy, the cells that are transfected or transduced in step i) of the method of sensitizing a cell to a nucleoside analogue prodrug may be transfected or transduced *in vivo, ex vivo or in vitro.*

Accordingly, in one embodiment, the method of sensitizing a cell to a nucleoside analogue prodrug defined above in the section "Summary of the invention" may be an *in vivo, ex vivo or in vitro* method, preferably *ex-vivo.*

The term "subject" herein refers to, for example, a human or a non-human mammal. For example, the subject is a bat; a ferret; a rabbit; a feline (cat); a canine (dog); a primate (monkey), an equine (horse); a human, including man, woman and child. In one embodiment a "subject" refers to a human.

In the context of the invention, the term "treating" or "treatment", refers to a therapeutic use (i.e. on a subject having a given disease) and means reversing, alleviating, inhibiting the progress of one or more symptoms of such disorder or condition. Therefore, treatment does not only refer to a treatment that leads to a complete cure of the disease, but also to treatments that slow down the progression of the disease and/or prolong the survival of the subject.

By "preventing" is meant a prophylactic use (i.e. on a subject susceptible of developing a given disease).

In one embodiment, a "disease" or "disorder" is any condition that would benefit from treatment with an isolated nucleic acid, an expression vector, a host cell or an isolated mutant hdCK of the invention or a pharmaceutical composition of the invention as defined above in the section "pharmaceutical composition".

In one embodiment the disease or disorder is cancer, a viral infection, Crohn's disease, a vascular condition, diabetes, Parkinson's disease, Huntington's disease, or Hurler syndrome, in particular cancer or a viral disease.

Accordingly, in one particular embodiment, the isolated nucleic acid, or expression vector or host cell or isolated mutant hdCK of the invention or pharmaceutical composition thereof are for use as a medicament for the treatment of cancer or for preventing a viral infection.

Accordingly, in a particular embodiment, the host cell of the invention or pharmaceutical composition thereof is for use as a medicament for the treatment of a disease or disorder selected from the group consisting of Crohn's disease, a vascular condition, diabetes, Parkinson's disease, Huntington's disease, or Hurler syndrome.

The term "in need of treatment" refers to a subject having already the disorder as well as those in which the disorder is to be prevented.

In a further embodiment, cancer relates to hematological cancer or solid tumors.

"Hematological cancers" or "hematological malignancies" refers to cancer that begins in blood-forming tissue, such as the bone marrow, or in the cells of the immune system. Examples of hematologic cancer are leukemia, lymphoma, and multiple myeloma. In particular, hematological cancers include leukemias (such as acute myelogenous leukemia, chronic myelogenous leukemia, acute lymphoid leukemia, chronic lymphoid leukemia, hairy cell leukemia and myelodysplasia syndrome) and malignant lymphoproliferative conditions, blastic plasmacytoid dendritic cell neoplasm (BPDCN), systemic mastocytosis, including lymphomas (such as multiple myeloma, non-Hodgkin's lymphoma, Burkitt's lymphoma, and small cell- and large cell-follicular lymphoma). In one embodiment, the hematological cancer is acute Myeloid leukaemia.

"Solid tumors" generally refers to an abnormal mass of tissue that usually does not contain cysts or liquid areas. Solid tumors may be benign (not cancer), or malignant (cancer). In one embodiment, the solid tumor is malignant. Different types of solid tumors are named for the type of cells that form them. Examples of solid tumors are sarcomas, carcinomas, and lymphomas). In one embodiment, the solid cancer is pancreatic carcinoma, non-small cell lung cancer, or relapsed ovarian cancer.

A "cancer cell" herein refers to a cell that is a hematological cancer cell or a solid tumor cell.

A "viral infection" herein refers to viral infections cause for example by herpesviruses, retroviruses (such as HIV) , orthomyxoviruses, hepatitis B and hepatitis C viruses, in particular herpesviruses or HIV-1.

In one embodiment, the subject has been diagnosed to suffer from cancer.

In a further embodiment, the subject has already been treated with chemotherapy until complete remission but relapsed.

The "relapse" is defined as the reoccurrence of AML after complete remission.

"Complete remission" or "CR" is defined as follows: normal values for neutrophil (>1.0*10⁹/L), haemoglobin level of 10g/dL and platelet count (>100*10⁹/L) and independence from red cell transfusion; blast cells less than 5%, no clusters or collections of blasts, and absence of Auer rods on bone marrow examination; and normal maturation of blood cells (morphology; myelogramme) and absence of extramedullary leukemia.

By a "therapeutically effective amount" of the isolated nucleic acid, a vector, an isolated mutant hdCK, or a host cell of the invention is meant a sufficient amount of the isolated nucleic acid, a vector, an isolated mutant hdCK, or a host cell of the invention to treat said cancer disease or to prevent said viral infection, at a reasonable benefit/risk ratio applicable to any medical treatment. Such effective amounts can be routinely determined by those of skilled in the art. It will be understood, however, that the amount of the compound actually administered will typically be determined by a attending physician within the scope of sound medical judgment. The specific therapeutically effective dose level for any particular subject will depend upon a variety of factors including the disorder being treated and the severity of the disorder; activity of the specific isolated nucleic acid, vector, isolated mutant hdCK, or host cell; the specific composition employed, the age, body weight, general health, sex and diet of the patient; the time of administration, route of administration; the duration of the treatment; drugs used in combination or coincidental with the specific isolated nucleic acid, vector, isolated mutant hdCK, or host cell employed; and like factors well known in the medical arts. It will also be appreciated by those of skilled in the art that the dosage may be dependent on the stability of the administered nucleic acid or vector.

The effective amount may also vary according to the drug or prodrug with which the nucleic acid may be co-administered.

### Imaging

Gene therapy is an attractive approach for treating different diseases such as SCID cystic fibrosis, hemophilia, Leber congenital amaurosis, muscular dystrophy, ornithine transcarbamylase deficiency, Pompe disease, and Gaucher's disease. A non-invasive imaging method is crucial to monitor gene therapies, the kinetics and the spatial distribution of the transgene expression as well as the population of cells comprising the transgene. Such imaging methods therefore enable investigators and physicians to monitor the safety of the therapy and eventually enables prognosis of the outcome.

"Imaging methods" herein refer to non-nuclear and nuclear imaging methods, preferably nuclear methods.

Non-nuclear imaging methods for example herein refer to magnetic resonance imaging (MRI) methods. In MRI the compound that that can be detected typically is a monocristalline iron oxide nanoparticle.

Another non-nuclear imaging method is the detection of bioluminescence by charged coupled devices (CCD) cameras. Said CCD cameras detect the photons that are emitted by bioluminescence.

Nuclear imaging methods include for example single photon emission computed tomography (SPECT) and positron emission tomography (PET).

In context of the present invention, transgene expression can be monitored indirectly using a reporter gene that is comprised in the transfected or transduced cell.

Accordingly, in one embodiment, the nucleic acid used for transfecting or transducing of a cell comprises a mutant hdCK, a further transgene and a reporter gene, or the nucleic acid used for transfecting or transducing of a cell comprises a nucleic acid comprising mutant hdCK and one nucleic acid comprising mutant hdCK, wherein one of the nucleic acids further comprises a reporter gene.

Accordingly, in another aspect, the invention provides a method of non-invasive imaging of transfected or transduced cells in a subject, and which method comprises the steps of
(i) contacting the cells in a subject with a detectable compound; and
(ii) non-invasively monitoring the quantity of said detectable compound in said cell or subject, wherein the cells in the subject comprise a nucleic acid comprising a mutant hdCK, a further transgene and a reporter gene or a nucleic acid comprising mutant hdCK and one nucleic acid comprising mutant hdCK, wherein the nucleic acid comprising mutant hdCK or nucleic acid comprising mutant hdCK further comprises a reporter gene.

Accordingly, in a further aspect, the invention provides a method of non-invasive imaging of transfected or transduced cells in a subject, and which method comprises the steps of
(i) contacting the cells in a subject with a detectable compound; and
(ii) non-invasively monitoring the quantity of said detectable compound in said cell or subject, wherein the cells in the subject comprise a nucleic acid comprising a mutant hdCK and a reporter gene or a nucleic acid comprising mutant hdCK and one nucleic acid comprising a reporter gene.

In one embodiment the reporter gene is a traceable marker such as GFP and the expression might be monitored upon expression of the nucleic acid comprising a mutant hdCK. Accordingly, in the same embodiment the method of non-invasive imaging comprises the steps of
i) non-invasively monitoring the quantity of the reporter gene in said cell or subject, wherein the cells in the subject comprise a nucleic acid comprising a mutant hdCK, a further transgene and a reporter gene or a nucleic acid comprising mutant hdCK and one nucleic acid comprising mutant hdCK, wherein the nucleic acid comprising mutant hdCK or nucleic acid comprising mutant hdCK further comprises a reporter gene.

Accordingly, in a further embodiment the method of non-invasive imaging comprises the steps of
ii) non-invasively monitoring the quantity of the reporter gene in said cell or subject, wherein the cells in the subject comprise a nucleic acid comprising a mutant hdCK and a reporter gene or a nucleic acid comprising mutant hdCK and one nucleic acid comprising a reporter gene.

In one embodiment, the reporter gene encodes an enzyme. Once the enzyme is expressed inside the cell it alters a detectable compound. The most common approach consists of the phosphorylation of a compound that can cross the plasma membrane of mammalian cells. The phosphorylated product becomes incapable of traversing the cell membrane and gets trapped inside the cell.

In a further embodiment, the reporter gene encodes a surface receptor. A detectable compound might bind to the receptor enabling detection by the imaging method.

In a further embodiment, the reporter gene encodes a transport protein having high specificity for certain compounds. Once expressed in cells said transporter uses active transport to concentrate the detectable compound enabling detection by the imaging method.

Reporter genes are as defined in the section "therapeutic methods".

In one embodiment, reporter genes in context of the invention are for example cytosine deaminase, HSV1-*tk*, D2R, mutant D2R, somatostatin receptor, Na/I symporter, luciferase, cathepsin D, metalloproteinase, *β*-galactosidase, mutated transferrin receptor, creatine kinase and arginine kinase.

It is known to the skilled in the art that each reporter genes requires a specific imaging agent, herein called detectable compound, and the corresponding imaging technology. Different reporter genes, detectable compounds and imaging methods used in gene therapy are for example described in the article Vasseau et al. (J Biomed Biotechnol. Apr 21, 2003; 2003(2): 92-101).

Accordingly, the reporter gene cytosine deaminase, for example, may be used in combination with the detectable compound is 5-[¹⁹F]fluorocytosine and Magnetic Resonance Spectroscopy (MRS) as imaging technology.

The reporter gene HSV1-*tk* might be used together with the detectable compound [¹³¹I]FIAU or [¹³¹I]FIAU when single photon emission-computed tomography (SPECT) is used, the detectable compound might be [¹⁴C]GCV, [³H]GCV for the same reporter gene in case of autoradiography and [¹⁸F]PCV or [¹⁸F]FHPG for positron emission tomography (PET).

In one embodiment the monitoring carried out in step (ii) is performed by Single Photon Emission Computed Tomography (SPECT), by Positron Emission Tomography (PET), by Magnetic Resonance Spectroscopy (MRS), by Magnetic Resonance Imaging (MRI), or by Computed Axial X-ray Tomography (CAT), or a combination thereof.

In a one embodiment the detectable compound is a labeled compound.

In one embodiment, the detectable compound is labeled with positron emitting radionuclides. All position emitting radionuclides are candidates for usage, the radionuclide is preferably selected from ²H (deuterium), ³H (tritium), ¹¹C, ¹³C, ¹⁴C, ¹⁵O, ¹³N, ¹²³1, ¹²⁵1, ¹³¹I₁ ¹⁸F and ^{99m} Tc.

An example of commercially available labeling agents, which can be used in the preparation of the labelled nucleoside analogue is [¹¹C]O₂, ¹⁸F, and Nal with different isotopes of Iodine. In particular [¹¹C]O₂ may be converted to a [¹¹C]-methylating agent, such as [¹¹C]H₃I or [¹¹C]-methyl triflate.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of"). Furthermore the indefinite article "a" or "an" does not exclude a plurality. The mere fact that certain measures are recited in mutually different dependent claims does not indicate that a combination of these measures cannot be used to advantage.

The invention will now be described in more details with reference to the following figures and examples. All literature and patent documents cited herein are hereby incorporated by reference. While the invention has been illustrated and described in detail in the foregoing description, the examples are to be considered illustrative or exemplary and not restrictive.

### BRIEF DESCRIPTION OF THE SEQUENCES

**SEQ ID NO: 1** shows the amino acid sequence of full-length hdCK as available from the NCBI database under accession number NP_000779.1.

**SEQ ID NO: 2** shows the nucleotide sequence of full-length hdCK corresponding to the nucleotides 41 to 823 of the human deoxynucleoside kinase mRNA sequence as available from the GenBank database under accession number JF806429.1.

**SEQ ID NO: 3** shows the amino acid sequence of the so called "G12" full-length mutant hdCK.

**SEQ ID NO: 4** shows the nucleotide sequence of the so-called "G12" full-length mutant hdCK.

**SEQ ID NO: 5** shows the amino acid sequence of the so called "M36" full-length mutant hdCK.

**SEQ ID NO: 6** shows the nucleotide sequence of the so-called "M36" full-length mutant hdCK.

**SEQ ID NO: 7** shows the amino acid sequence of mutant hdCK having the mutation S169N in comparison to the wild type sequence.

**SEQ ID NO: 8** shows the amino acid sequence of mutant hdCK having the mutations S169N and E171 K in comparison to the wild type sequence.

**SEQ ID NO: 9** shows the amino acid sequence of mutant hdCK having the mutations S169N and E247K in comparison to the wild type sequence.

**SEQ ID NO: 10** shows the amino acid sequence of mutant hdCK having the mutations S169N and L249M in comparison to the wild type sequence.

**SEQ ID NO: 11** shows the amino acid sequence of mutant hdCK having the mutations S169N, E171 K and E247K in comparison to the wild type sequence.

**SEQ ID NO: 12** shows the amino acid sequence of mutant hdCK having the mutations S169N, E171 K and L249M in comparison to the wild type sequence.

**SEQ ID NO: 13** shows the amino acid sequence of mutant hdCK having the mutations S169N, E247K and L249M in comparison to the wild type sequence.

### FIGURES

**Figure 1****.** Graph representing the sensitivity to gemcitabine of Messa10K cells induced by M36. The percentage of alive cells over the total amount of cells is plotted as a function of the concentration of gemcitabine. Diamonds, Messa 10K cells; triangles wt dCK; squares G12; circles M36.
**Figure 2****.** Graph representing the sensitivity to gemcitabine of Messa10K cells induced by M36 to a wider range of gemcitabine than used in Figure 1. Diamonds, Messa 10K cells; triangles wt dCK; squares G12; circles M36.
**Figure 3****.** Schematic representation of the localisation of the mutations characterising M36 in the human dCK. The position of the first and of the last amino acids are indicated. The location and numbering of the alpha helixes and of the beta sheets is given in green/light grey and blue/dark grey, respectively. The position and nature of the four mutations present in M36 is given below the drawing.
**Figure 4****.** Schematic representation of the experimental procedure used to test the level of resistance to infection by HIV-derived vectors of cells expressing constitutively M36 and treated in the presence of the anti-HIV compounds ddC and 3TC.
**Figure 5****.** Graph representing the sensitivity to infection of HEK 293T / CD4+ cells to HIV in the presence of varying concentrations of ddC of cells encoding M36 (black circles) or an extra copy of the wt dCK gene (white circles).
**Figure 6****.** Graph representing the sensitivity to infection of HEK 293T / CD4+ cells to HIV in the presence of varying concentrations of 3TC (Lamivudine) of cells encoding M36 (black circles) or an extra copy of the wt dCK gene (white circles).
**Figure 7****.** Graph representing the sensitisation to anti cancer compound other than gemcitabine obtained by M36. The percentage of alive cells over the total amount of cells is plotted as function of the concentration of the anticancer compound. White circles, Messa 10K cells; grey diamonds, wt dCK; black squares, M36. *Panel A,* sensitivity to Fludarabine. *Panel B,* sensitivity to AraC. The x axis is given in log scale.
**Figure 8****.** Graphs representing the biochemical comparative characterisation of phosphorylation of AraC by wt dCK and M36. Phsphorylation kinetics of wt dCK (grey circles) and M36 (white circles) overexpressed in *E. coli.* Steady state kinetic data are fitted according to the Michaelis-Menten equation. *Panel A,* phosphorylation of dC. *Panel B,* phosphorylation of gemcitabine. *Panel C,* phosphorylation of AraC.
**Figure 9****.** Tables representing the biochemical comparative characterisation of phosphorylation of AraC by wt dCK and M36. *Panel A,* ratio of 1/Km and of Kcat for M36 vs wt dCK, with respect to the natural substrate dC (black), to gemcitabine (grey), and to AraC (white). *Panel B,* ratio of Km (black) and of Kcat (grey) for M36 vs G12, with respect to the gemcitabine and dC. The dotted line gives the reference of a ratio of 1. **EXAMPLE**

### Materials and Methods

**Tests of sensitivity to anticancer drugs.** Cells were seeded at 5000 cells per well in a 96-well plate and grown overnight at 37°C. Two rows were used for each population. 12h after seeding, increasing concentrations of Gemcitabine (0-400 nM or 0-100µM), or AraC (0-10 mM) were added to each well and left in culture for further 72h. Cell viability was measured by MTT test (CellTiter 96 Non-Radioactive Cell Proliferation Assay, Promega) and the number of living cells in each well was evaluated by measuring the OD at 570 nm. For each population, the fraction of alive cells was calculated as OD570 concentration X Gem/OD570 concentration 0 Gem, to estimate the sensitivity of the population to the prodrug.

**Tests of sensitivity to antiviral drugs.** Viral vectors were generated by transfection of HEK-293T cells with 10 µg of pCMVΔR8.91 plasmid, 5 µg of pHCMV-G plasmid, and 10 µg of genomic plasmid SDY-dCK (described in Rossolillo et al. 2012) encoding either for the wild type sequence of the human dCK or the M36 variant. Transduction was performed on 1×10⁶ HEK-293T-CD4+ cells with viral vectors. Transduced cells were selected in the presence of 0.6 µg/ml of puromycin, added 24h after transduction. 50 to 200 individual puromycin-resistant clones were pooled and expanded obtaining the polyclonal wt-dCK cell population (wtdCK-pcP, for wt dCK polyclonal population) and the M36 wt-dCK cell population (M36-pcP). Vectors for challenging infection in the presence of antiviral drugs were generated by transfection with 10 µg of pTopo plasmid encoding for the HIV envelope ADA (ref, plus Hamoudi 2013), 5 µg of pHCMV-G plasmid, and 10 µg of genomic plasmid SDY-Luc, which is a variant of the pSDY plasmid encoding for the firefly luciferase. These viruses were used to transduce 1×10⁶ HEK-293T-CD4+-wtdCK or HEK-293T-CD4+-M36 cells. Viral infection was then monitored 48 hours after transduction. For this the medium was removed, the cells were washed twice in PBS, lysed, centrifuged, and the supernatant was used to measure luciferase activity according to the manufacturer's protocol (Promega, Fitchburg, WI, USA) with a Glomax luminometer (Promega, Fitchburg, WI, USA).

**Western blot analyses.** Messa10K cell lines expressing either the wt dCK or M36, both in the context of a proviral DNA with functional LTR or with an inactivated LTR were lysed in 1X RIPA buffer, and 30, 60, 120 µg of total protein (evaluated by Bradford) for each cell type and loaded on a 12% bis-tricine gel (Invitrogen). After transfer on a PVDF membrane, the dCK proteins were analysed by western Blot with 1:4000 dilution of a polyclonal anti-dCK antibody (rabbit, Sigma-Aldrich) and 1:3000 anti-rabbit HRP (BioRad) conjugated secondary antibody and detected by autoradiography.

**Production and purification of recombinant dCK proteins.** The wt-dCK and M36 sequences were cloned in the pET14b plasmid and expressed in *E. coli cells* BL21 DE3 pLysE. Protein expression was induced by adding 0.1 mM IPTG, and cells were collected after 4h of growth at 37°C. His-tagged proteins were eluted with 250 mM Imidazole from His-Trap TM FF Columns (GE HealthCare), the Histidine tag was removed using the STag Thrombin Purification Kit (Novagen), and dCK and G12 were further purified by gel filtration on S-200 Sephacryl columns (GE Healthcare).

**Phosphorylation tests in vitro.** The efficiency of phosphorylation of the natural substrate deoxycytidine and of the prodrugs gemcitabine and AraC were measured for purified wt-dCK and M36 in a NADH-based assay as previously described. All reagents were purchased from Sigma (France) except Gemcitabine (Lilly France SAS). Enzymes were assayed at RT at a concentration of 0.9 µM for dC, 0.3 µM for Gemcitabine, and X for AraC. dC was used at concentrations between 5 and 50 µM, Gemcitabine at concentrations between 10 µM and 1mM, and AraC. ATP was 4 mM. All experiments were performed in triplicate.

### Results

### Comparison of wild-type hdCK and G36 and G12 mutant hdCK

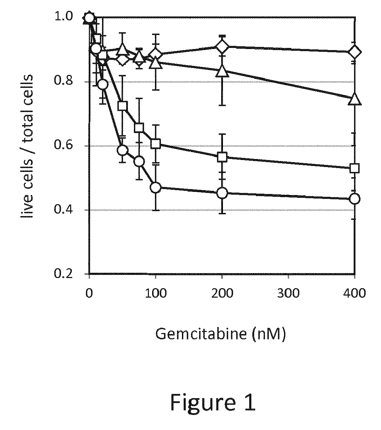

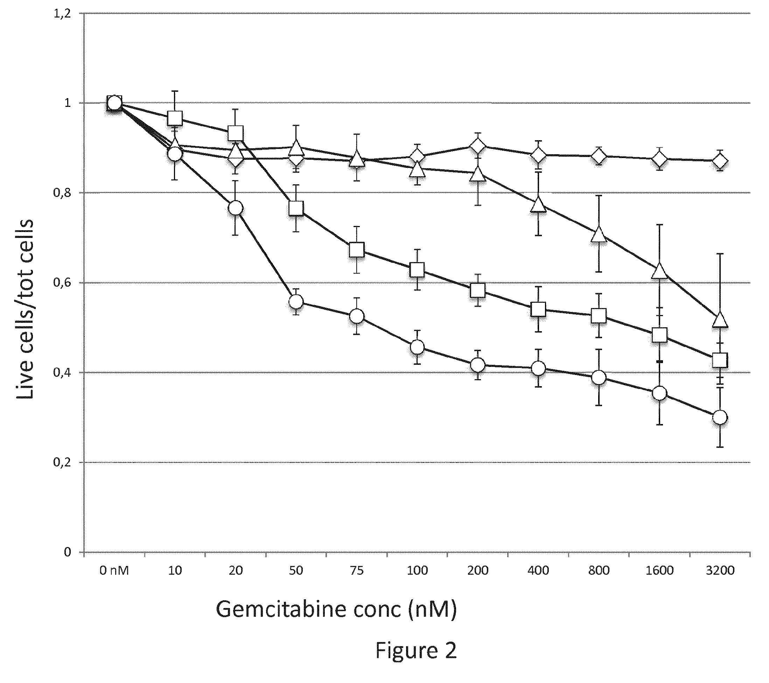

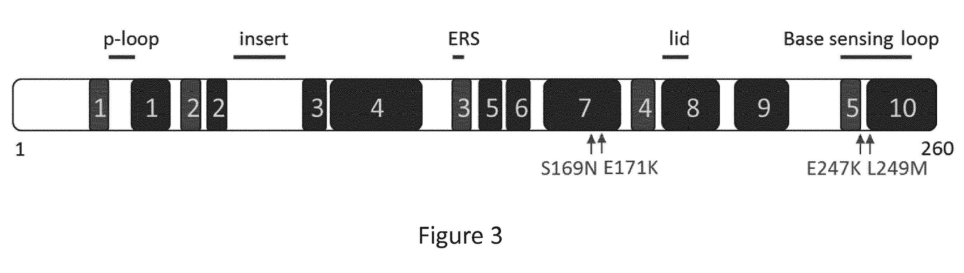

Messa10K cells were induced with a wild-type hdCK viral vector, a G12 hdCK viral vector and G36 hdCK viral vector. Briefly, after transduction with the viral vectors the different Messa 10K populations were seeded into 96-well dishes, exposed to varying concentrations of Gemcitabine, and cell survival was measured by an MTT test as previously described (Rossolillo et al. 2012). Surprisingly, the M36 mutant hdCK demonstrated a further improved sensitisation phenotype compared to G12 (Figure 1). After the first comparative experiment the phenotype conferred by M36 was confirmed and compared to the G12 one in experiments with a wider range of gemcitabine concentrations (Figure 2). With respect to G12, M36 contains an additional mutation (S169N), located in the vicinity one of the mutations present in G12 (E171K). M36 therefore presents four mutations clustered in pairs in the 247-249 (E247K, L249M) and 169-171 region (S169N, E171 K) (Figure 3).

### Characterisation of M36 in cell culture with a ΔLTR lentiviral vector

The use of SIN vectors is required for biomedical applications. To this end M36 has been inserted in a SIN version of pSDY, and the comparison between efficiency of M36 and G12 has been done in polyclonal populations of Messa 10K cells generated by transduction with M36 or with G12 SIN vectors. The difference between the structure of the proviral DNA generated with these two types of vector is the presence of a functional or inactivated (for SIN vectors) promoter to drive the expression of the integrated genomic RNA. Also using SIN vectors M36 was more efficient than G12 with a reduction of the IC50 from 75 nM to 20 nM. In parallel, with respect to wt LTR, a higher proportion of cells was induced to death, since the plateau of maximum mortality observed was set at 80% of death instead of the 60%. Finally, for both variants, G12 and M36, the use of SIN vectors appeared more efficient than that of wt vectors (data not shown).

In order to understand whether the enhanced effect observed with the SIN vectors could be due to a higher production of M36 in the presence of a ΔLTR variant, we investigated whether an increased production of M36 was observed in Messa10K cells encoding the M36 in the context of a proviral DNA carrying a functional LTR or a its partially deleted counterpart. An increased amount of M36 was detected by Western blot analyses from cells harbouring a provirus with an inactive LTR, possibly accounting for the increased sensitization observed with M36 in these cells (Data not shown).

### M36 and sensitisation to antiviral and other anticancer compounds

The biochemical characterisation of M36, the increased sensitivity of Messa 10K cells to gemcitabine treatment is mostly due to a decrease in the ability to phosphorylate the natural substrate (dC) while maintaining a good level of phosphorylation of the gemcitabine. This is expected to lead to a reduced competition between dC and the drug, inside the cell. We reasoned that, if the ability to phosphorylate other drugs were retained in the mutant, the decreased phosphorylation of dC should also enhance sensitisation to other drugs. Two antiviral and two anti-cancer drugs that are activated through phosphorylation by dCK were tested, 3TC and ddC, and Fludarabine and AraC, respectively.

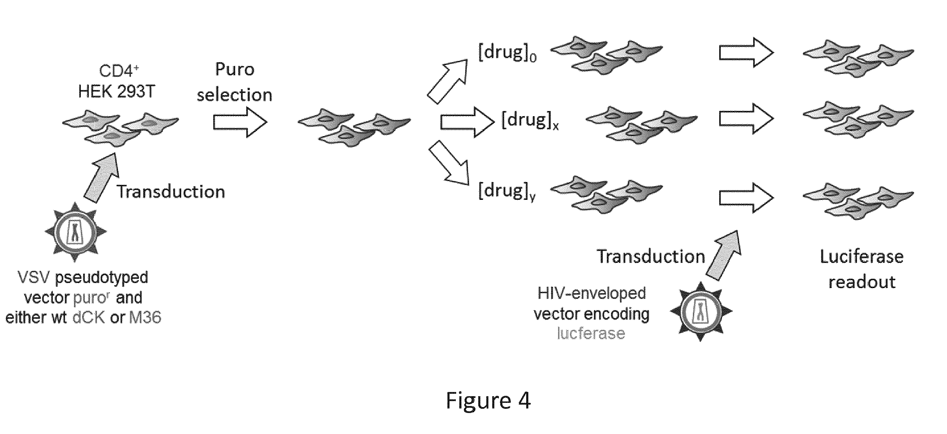

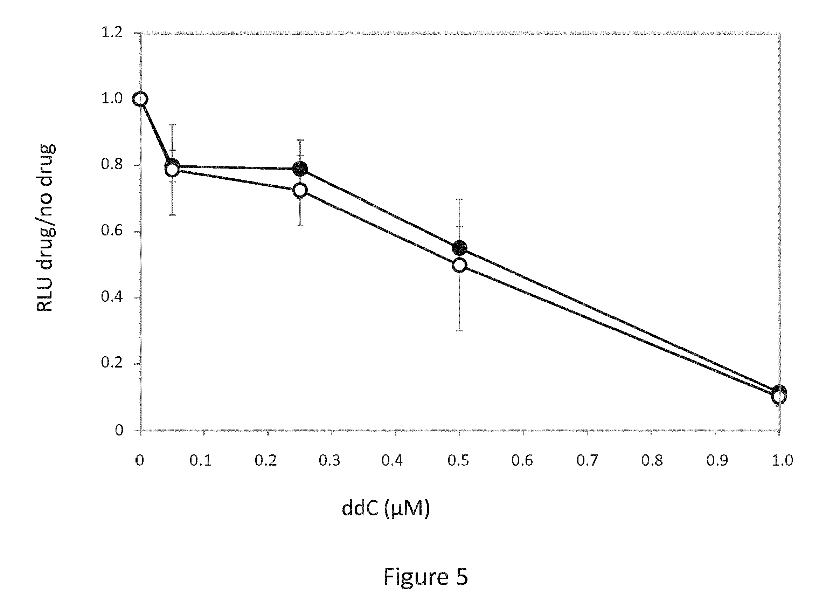

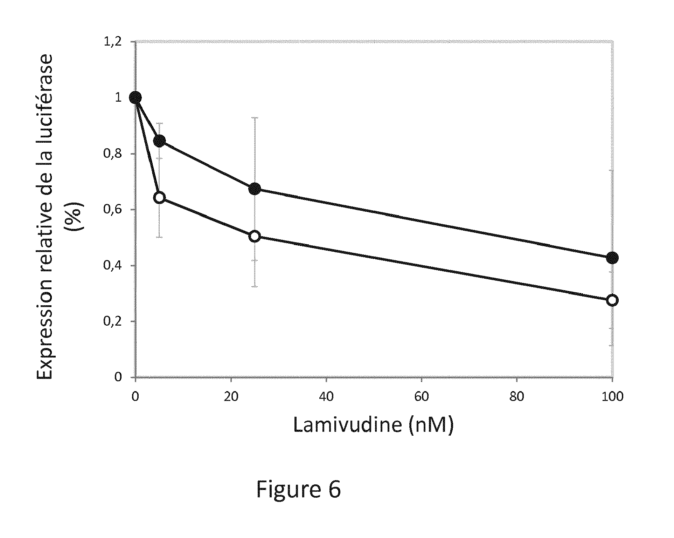

For the antiviral test, the following assay was developped es demonstrated in Figure 4. Firstly HEK 293T/CD4⁺ cells were transduced at a low MOI of by the vector encoding the M36 variant and selection of the successfully transduced cells was perdfomed using puromycin. The selected cell line was then transduced with lentiviral vectors, as described in Materials and Methods, encoding the luciferase gene and luciferase levels ere measured 48 hours after infection. A comparison with the level of luciferase detected in the parallel infection of control HEK 293T/CD4⁺ cells expressing as a transgene the wt dCK gene was performed and the data plotted. As shown in the figure, no significant increase in resistance was observed with ddC (Figure 5), while a lower infectivity (in the 2-fold range) was observed for 3TC (Figure 6).

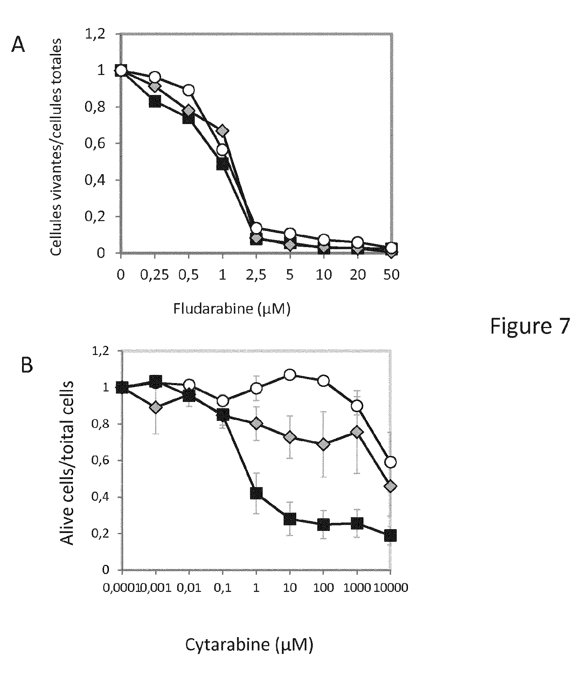

The test with the anticancer drugs was performed as previously described, for gemcitabine. The results were markedly different with the two compounds tested. If, in the case of fludarabine, no significant sensitisation by M36 of Messa 10K cells was observed (Figure 7A), in sharp contrast, for AraC, a strong sensitisation is observed in M36-expressing cells with respect to control cells expressing an exogenous copy of the wt dCK gene (Figure 7B). In this case, a decrease in the IC50 of approximately 10 000 times was observed for cells containing M36 with respect to cells containing the wt dCK as exogenous gene.

### Biochemical characterisation of phosphorylation of gemcitabine and AraC by M36

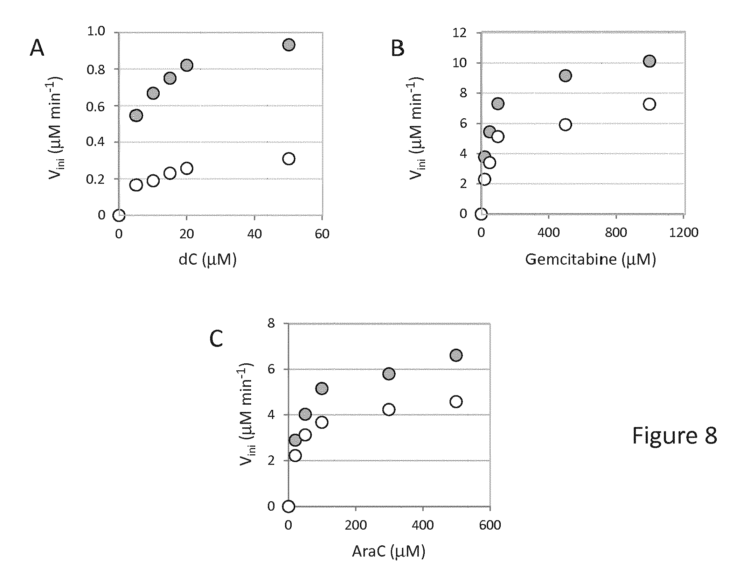

To characterise its biochemical properties M36 and, as a control, the wt dCK protein, were produced as recombinant, C-ter His tagged proteins in *E. coli,* purified and tested for their efficiency of phosphorylation in vitro. As it was the case for the G12 mutant (Rossolillo et al. 2012) also for M36 the substitutions present in the mutant led to a decrease in the Vₘₐₓ of phosphorylation as function of the concentration of substrate, for both dC and for gemcitabine, but while the decrease is dramatic for the dC it is only modest for gemcitabine (Figure 8A-B). This observation is strictly similar to what seen with G12 (Rossolillo et al 2012) suggesting that, also for M36, the balance between phosphorylation of dC with respect to the gemcitabine is more in favour of the drug than for the wild type dCK. The results obtained with AraC follow the same trend, with a very modest reduction of the Vₘₐₓ of phosphorylation as function of the concentration of this substrate for M36, compared to what observed for wt dCK (Figure 8C).

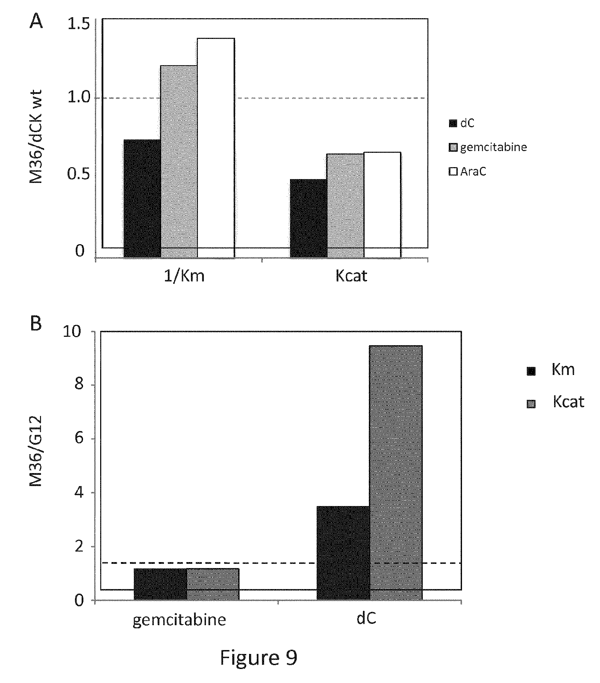

A comparison of Km and Kcat values for dC, gemcitabine and AraC found with M36 and with wt dCK shows that with M36 the values of Kcat decrease to approximately 50% those of wt dCK, for the three substrates (Figure 9A). Differences between the natural substrate and gemcitabine or AraC were instead observed when considering Km values. Indeed an increased Km is observed for gemcitabine and AraC (higher 1/Km values in Figure 9A) while, for the natural substrate, the situation is reversed. This observation suggests that the sensitisation observed in cell culture is due to an increased relative affinity of the mutant for both anticancer compounds and a parallel decreased relative affinity for the natural substrate.

In order to address why, despite the overall similarity of the biochemical properties of M36 and G12, M36 induced a stronger sensitisation to Gemcitabine than G12, Km and Kcat values for dC and gemcitabine were also compared between M36 and G12, by dividing the values found with M36 by those reported for G12 by Rossolillo and colleagues (Rossolillo et al. 2012). For gemcitabine, both parameters were strictly comparable between the two mutants (Figure 9B), with a ratio of 1 (dotted line). For the natural substrate, instead, an increase both in Km (by a 3.5 factor) and in Kcat (by a 9.5 factor) was observed for M36. This implies that this mutant has a lower affinity for the natural substrate but an improved catalytic activity. The more efficient sensitisation induced by M36 with respect to G12 in cell culture, thereby suggests that, within the cell, the affinity for the substrate is more important than the catalytic activity itself.

### Discussion

Suicide gene therapy is an approach of potential interest in many fields as clinical treatment of cancers with poor prognosis and as security gene in cell therapy approaches. We previously described the identification of a triple mutant of the human dCK gene that sensitises a panel of cancer cells (HEK 293T, Messa 10K, and BxPC3) to treatment with low doses of gemcitabine. Here a new mutant was identifdied that leads to an improved sensitisation to gemcitabine. Furthermore, the M36 mutant not only increases sensitivity to gemcitabine but also to AraC, another anticancer compound currently used in clinics. The sensitisation to AraC was even much stronger than what observed for gemcitabine. In addition, we report that the use of SIN lentiviral vectors further magnifies the effect sensitisation observed.

The biochemical characterisation of M36 depicts a similar pattern of enzymatic properties compared to G12, with a more drastic decrease in the efficiency of phosphorylation of the natural substrate than for gemcitabine when comparing M36 to wt dCK. A comparison between the values found here for M36 and those reported for G12 suggests, interestingly, that the parameter that can account for the improved sensitisation to gemcitabine observed in cell culture with M36 with respect to G12 is an increased affinity for the drug. The lower efficiency of phosphorylation per se observed with M36 with respect to G12 does not seem to be critical *in vivo,* though.

Interestingly the use of the SIN vectors further sensitises cells. It is further shown here that this is probably due to relieved transcriptional interference occurring when the U3 promoter is functional. This translates in a higher amount of M36 synthesised that is expected to increase the intracellular concentration of activated drug. The loss of the potential enhancing effect exerted by the functional LTR on the expression of the neighbouring sequences, including the internal promoter EF1-α that drives the expression of M36, does not seem to impact the amount of M36 protein present in the cell, instead.

## Claims

1. An isolated nucleic acid comprising a sequence encoding a mutant human deoxycytidine kinase (hdCK), wherein said mutant hdCK comprises a polypeptide sequence which differs from wild-type hdCK of sequence SEQ ID NO: 1 by at least one mutation, wherein said at least one mutation is at the amino acid position S169, in comparison to said amino acid sequence SEQ ID NO: 1.

2. The isolated nucleic acid according to claim 1, wherein said mutant hdCK comprises a polypeptide sequence which differs from wild-type hdCK of sequence SEQ ID NO: 1 by at least one further mutation, wherein the at least one further mutation is at the position E171, E247 or L249, in comparison to the amino acid sequence SEQ ID NO: 1.

3. The isolated nucleic acid according to claim 1 or 2, wherein the encoded mutated hdCK increases the phosphorylation of a nucleoside analogue.

4. The isolated nucleic acid according to any one of claims 1 to 3, which comprises a nucleotide sequence encoding a mutant hdCK of sequence SEQ ID NO: 5, 7, 8, 9, 10, 11, 12 or 13, or a variant thereof.

5. An isolated expression vector comprising the nucleic acid according to any one of claims 1 to 4.

6. An isolated host cell genetically engineered with the vector according to claim 5.

7. A process for producing a mutant human deoxycytidine kinase (hdCK) comprising *in vitro* culturing a host cell of claim 6 and recovering the expressed mutant hdCK from the cultured host cells and/or culture medium.

8. An isolated mutant human deoxycytidine kinase (hdCK) comprising a polypeptide sequence which differs from wild-type hdCK of sequence SEQ ID NO: 1 by at least one mutation, wherein the at least one mutation is at the amino acid position S169 in comparison to the amino acid sequence SEQ ID NO: 1.

9. A pharmaceutical composition comprising the isolated nucleic acid according to any one of claims 1 to 4, the expression vector according to claim 5, the host cell according to claim 6, or the isolated mutant hdCK according to claim 8 and a pharmaceutically acceptable carrier or diluent.

10. The isolated nucleic acid according to any one of claims 1 to 4, the expression vector according to claim 5, the host cell according to claim 6, the isolated mutant hdCK according to claim 8, or a pharmaceutical composition according to claim 9 for use as a medicament.

11. The isolated nucleic acid according to any one of claims 1 to 4, the expression vector according to claim 5, the host cell according to claim 6, the isolated human mutant deoxycytidine kinase according to claim 8, or a pharmaceutical composition according to claim 9 for use for the treatment of cancer or for the prevention of a viral infection.

12. A kit comprising a pharmaceutical composition according to claim 9 and a nucleoside analogue.

13. An *ex-vivo* method of sensitizing a cell to a nucleoside analogue prodrug, which method comprises the steps of:
(i) transfecting or transducing cells with a nucleic acid of any of claims 1 to 4, or with an expression vector according to claim 5.

14. A nucleic acid according to any one of claims 1 to 4 for use as a safety gene in gene therapy or cell therapy.

15. A method of non-invasive imaging of transfected or transduced cells in a subject, and which method comprises the steps of
(i) contacting the cells in a subject with a detectable compound; and
(ii) non-invasively monitoring the quantity of said detectable compound in said cell or subject, wherein the cells in the subject comprise a nucleic acid comprising a mutant hdCK, a further transgene and a reporter gene or a nucleic acid comprising mutant hdCK and one nucleic acid comprising mutant hdCK, wherein the nucleic acid comprising mutant hdCK or nucleic acid comprising mutant hdCK further comprises a reporter gene.
